(19) Europäisches Patentamt
European Patent Office
Office européen des brevets

(11) Veröffentlichungsnummer: **0 102 929**
**B1**

(12) # EUROPÄISCHE PATENTSCHRIFT

(45) Veröffentlichungstag der Patentschrift: **01.07.87**

(21) Anmeldenummer: **83810398.4**

(22) Anmeldetag: **01.09.83**

(51) Int. Cl.[4]: **C 07 C 93/00,** A 61 K 31/135, C 07 C 149/42, C 07 D 295/08, C 07 C 147/14, C 07 C 93/14 // C07C101/04, C07C91/04, C07C69/65, C07C125/065, C07C99/00

(54) **Propylamin-Derivate, Verfahren zu ihrer Herstellung, pharmazeutische Präparate enthaltend diese Verbindungen, sowie ihre therapeutische Verwendung.**

(30) Priorität: **07.09.82 US 415000**

(43) Veröffentlichungstag der Anmeldung: **14.03.84 Patentblatt 84/11**

(45) Bekanntmachung des Hinweises auf die Patenterteilung: **01.07.87 Patentblatt 87/27**

(84) Benannte Vertragsstaaten: **AT BE CH DE FR GB IT LI LU NL SE**

(56) Entgegenhaltungen:
**FR-A-2 034 453**
**FR-A-2 148 001**
**GB-A-2 054 588**

(73) Patentinhaber: **CIBA- GEIGY AG, Klybeckstrasse 141, CH- 4002 Basel (CH)**

(72) Erfinder: **Watthev, Jeffrey W.H., Dr., 61 Deepwood Drive, Chappaque New York 10514 (US)**

EP 0 102 929 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die französische Patentanmeldung 2,148,001 [und auch die im wesentlichen äquivalenten kanadischen, belgischen und britischen (975,375; 786,450 bzw. 1,343,527) Texte] betrifft solche Verbindugen welche als gewisse 2-(Aryl, Aralkinyl oder Aralkyl)-3-(aryl oder alk)-oxypropylamine bezeichnet sind. Diese Verbindungen haben eine Wirkung auf das kardiovaskuläre System. Im Beispiel 60 ist eine Verbindung gezeigt worden deren Struktur dem 2-Benzyl-3-phenoxy-N,N-diethyl-propylamin entspricht. Bei der Wiederholung des Verfahrens des Beispiels 60 der französischen Anmeldung 2,148,001 hatte man nun gefunden, dass das Produkt des Beispiels 60 eigentlich in wesentlichen aus einer Verbindung besteht, welche die zugeschriebene Struktur nicht hat. Anstelle der angenommenen hat diese Verbindung die Struktur des 3-Phenoxy-methyl-3-phenyl-N,N-diethyl-propylamins,

Der Erfindung liegt die Aufgabe zugrunde, ein neues Arzneimittel mit Wirkung auf das zentrale Nervensystem zu schaffen das die Hemmung der Serotonin-Aufnahme der Gehirn-Nervenzellen bewirkt.

Die Aufgabe wird erfindungsgemäss durch die Bereitstellung der neuen Propylamin-Verbindungen der Formel I gelöst.

Die Erfindung betrifft neue Verbindungen der allgemeinen Formel I

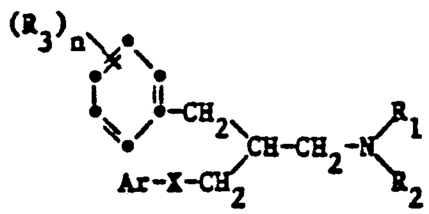

(I),

worin jedes der Symbole $R_1$ und $R_2$ Wasserstoff oder Niederalkyl bedeutet, oder $R_1$ und $R_2$ zusammen mit des Stickstoffatom fur Piperidino oder Pyrrolidino, oder für einen fünf- oder sechsgliedrigen gesättigten heterocyclischen Ring stehen, der NH, N-Niederalkyl, O oder S enthält; $R_3$ Wasserstoff, Niederalkyl, Niederalkoxy, Halogen oder Trifluormethyl bedeutet; Ar für gegebenenfalls durch einen bis drei Substituenten der Gruppe Niederalkyl, Niederalkoxy, Niederalkenoyloxy, Niederalkinyloxy, Niederalkylmercapto, Niederalkylsulfinyl, Niederalkylsulfonyl, Halogen und Trifluormethyl, oder durch ein Niederalkylendioxy substituiertes Phenyl steht; X ein Sauerstoffatom, Schwefelatom oder N-Niederalkyl bedeutet, und n für die ganze Zahl 1, 2 oder 3 steht, ihre Säureadditionssalze, insbesondere ihre therapeutisch verwendbaren Säureadditionssalze; ihre N-Oxide und ihre N-Niederalkyl oder N-Benzyl quaternären Salze, mit Ausnahme der Verbindung der Formel I, worin jedes der Symbole $R_1$ und $R_2$ Ethyl bedeutet, $R_3$ für Wasserstoff steht, Ar Phenyl bedeutet, X für ein Sauerstoffatom steht, und n 3 bedeutet, wobei die mit "nieder" bezeichneten Gruppen höchstens 7 Kohlenstoffatome enthalten, und ihrer Salze; sowie erfahren zu ihrer Herstellung, pharmazeutische Präparate enthaltend diese Verbindungen, sowie ihre therapeutische Verwendung.

Insbesondere betrifft die vorliegende Erfindung Verbindungen der allgemeinen Formel I, worin jedes der Symbole $R_1$ und $R_2$ Wasserstoff oder Niederalkyl bedeutet, oder $R_1$ und $R_2$ zusammen mit dem Stickstoffatom für Piperidino oder Pyrrolidino, oder für einen fünf- oder sechsgliedrigen gesättigten heterocyclischen Ring stehen, der NH, N-Niederalkyl, O oder S enthält; $R_3$ Wasserstoff, Niederalkyl, Niederalkoxy, Halogen oder Trifluormethyl bedeutet; Ar für gegebenenfalls durch einen bis drei Substituenten der Gruppe Niederalkyl, Niederalkoxy, Niederalkenyloxy, Niederalkinyloxy, Niederalkylmercapto, Niederalkylsulfinyl, Niederalkylsulfonyl, Halogen und Trifluormethyl, oder durch ein Niederalkylendioxy substituiertes Phenyl steht; X ein Schwefelatom oder N-Niederalkyl bedeutet, und n für die ganze Zahl 1, 2 oder 3 steht; wobei die mit "nieder" bezeichneten Gruppen höchstens 7 Kohlensstoffatome enthalten, ihre Säureadditionssalze, insbesondere ihre therapeutisch verwendbaren Säureadditionssalze; ihre N-Oxide und ihre N-Niederalkyl oder N-Benzyl quaternären Salze.

Bevorzugt sind Verbindungen der allgemeinen Formel I, worin $R_1$ und $R_2$ unabhängig voneinander Wasserstoff oder Niederalkyl mit 1-7 Kohlenstoffatomen bedeuten, oder $R_1$ und $R_2$ zusammen mit dem Stickstoffatom für Pyrrolidino oder Piperidino stehen; $R_3$, Ar und X die oben angegebenen Bedeutungen haben, und ihre Säureadditionssalze, insbesondere ihre therapeutisch verwendbaren Salze, mit Ausnahme der Verbindung der Formel I, worin jedes der Symbole $R_1$ und $R_2$ Ethyl bedeutet, $R_3$ für Wasserstoff steht, Ar Phenyl bedeutet, X für ein Sauerstoffatom steht und n 3 bedeutet und ihrer Salze.

Als Illustration der oben genannten bevorzugten Verbindungen sind zu nennen solche der allgemeinen Formel I, worin $R_1$ und $R_2$ unabhängig voneinander Wasserstoff, Methyl oder Ethyl bedeuten; $R_3$ für Wasserstoff, Chlor oder Trifluormethyl steht, Ar gegebenenfalls durch einen oder zwei Substituenten der Gruppe Methyl, Methoxy, Allyloxy, Methylthio, Methylsulfinyl, Chlor, Brom oder Trifluormethyl oder durch ein Methylendioxy substituiertes Phenyl bedeutet; X für O oder S steht, und ihre Säureadditionssalze,

insbesondere therapeutisch verwendbaren Salze, mit Ausnahme der Verbindung der Formel I, worin jedes der Symbole $R_1$ und $R_2$ Ethyl bedeutet, $R_3$ für Wasserstoff steht, Ar Phenyl bedeutet, X für ein Sauerstoffatom steht, und n 3 bedeutet, und ihrer Salze.

Sehr bevorzugt sind Verbindungen der Formel I, worin $R_1$ und $R_2$ unabhängig voneinander Wasserstoff oder Niederalkyl bedeuten, oder $R_1$ und $R_2$ zusammen mit dem Stickstoffatom Pyrrolidino oder Piperidino bedeuten oder für einen fünf- oder sechsgliedrigen gesättigten heterocyclischen Ring stehen, der NH, N-Niederalkyl, O oder S enthält, $R_3$ Wasserstoff, Niederalkyl, Niederalkoxy, Halogen oder Trifluormethyl bedeutet, n für die ganze Zahl 1 oder 2 steht, X Sauerstoff, Schwefel oder N-Niederalkyl bedeutet, Ar für einen Phenylrest steht, der durch einen oder zwei Substituenten der Gruppe Niederalkyl, Niederalkoxy, Niederalkenyloxy, Niederalkinyloxy, Niederalkylmercapto, Niederalkylsulfinyl, Niederalkylsulfonyl, Halogen und Trifluormethyl, oder durch ein Niederalkylendioxy substituiert ist, und ihre Säureadditionssalze, insbesondere ihre therapeutisch verwendbaren Säureadditionssalze.

Die Erfindung betrifft insbesondere Verbindungen der Formel IA

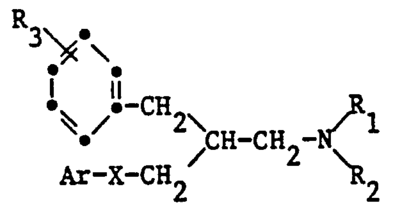

(IA),

worin $R_1$ und $R_2$ unabhängig voneinander Wasserstoff oder Niederalkyl bedeuten, oder $R_1$ und $R_2$ zusammen mit dem Stickstoffatom für Piperidino oder Pyrrolidino, oder für einen fünf- oder sechsgliedrigen gesättigten heterocyclischen Ring stehen, der NH, N-Niederalkyl, O oder S enthält; $R_3$ Wasserstoff, Niederalkyl, Niederalkoxy, Halogen oder Trifluormethyl bedeutet; Ar für einen Phenylrest steht, der durch einen oder zwei Substituenten der Gruppe Niederalkyl, Niederalkoxy, Niederalkenyloxy, Niederalkinyloxy, Halogen oder Trifluormethyl oder durch ein Niederalkylendioxy substituiert ist, und X für O oder S steht; wobei die mit "nieder" bezeichneten Gruppen höchstens 7 Kohlenstoffatome erhalten, ihre Säureadditionssalze, insbesondere ihre therapeutisch verwendbaren Säureadditionssalze; ihre N-Oxide und N-Niederalkyl oder N-Benzyl quaternären Salze.

Bevorzugt sind weiter Verbindungen der Formel IA worin $R_1$ und $R_2$ unabhängig voneinander Wasserstoff oder Niederalkyl mit 1 bis 7 Kohlenstoffatomen bedeuten, oder $R_1$ und $R_2$ zusammen mit dem Stickstoffatom für Pyrrolidino oder Piperidino stehen; $R_3$, Ar und X die vorher angegebenen Bedeutungen haben; und ihre Säureadditionssalze, insbesondere ihre therapeutisch verwendbaren Salze.

Besonders bevorzugt sind Verbindungen der Formel IA, worin $R_1$ und $R_2$ unabhängig voneinander Wasserstoff oder Niederalkyl bedeuten, oder $R_1$ und $R_2$ zusammen mit dem Stickstoffatom für Piperidino stehen; $R_3$ Wasserstoff, Halogen oder Trifluormethyl bedeutet; Ar für Phenyl steht, das durch einen oder zwei, gleiche oder verschiedene Substituenten der Gruppe Niederalkyl, Niederalkoxy, Niederalkenyloxy, Niederalkylmercapto, Niederalkylsulfinyl, Niederalkylsulfonyl, Halogen oder Trifluormethyl, oder durch ein Niederalkylendioxy substituiert ist, wobei die mit "nieder" bezeichneten Gruppen höchtens 7 Kohlenstoffatome enthalten; und X Sauerstoff oder Schwefel bedeutet, und ihre Säureadditionssalze, insbesondere ihre therapeutisch verwendbaren Salze.

Als Beispiele für die vorher genannten bevorzugten Verbindungen seien diejenigen der allgemeinen Formel IA genannt, in welchen $R_1$ und $R_2$ unabhängig voneinander Wasserstoff, Methyl oder Ethyl bedeutet; $R_3$ für Wasserstoff, Chlor oder Trifluormethyl steht; Ar Phenyl bedeutet, welches durch einen oder zwei Substituenten der Gruppen Methyl, Methoxy, Allyloxy, Methylmercapto, Methylsulfinyl, Chlor, Brom oder Trilfuormethyl oder durch ein Methylendioxy substituiert ist, X für Sauerstoff oder Schwefel steht, und ihre Säureadditionssalze, insbesondere therapeutisch verwendbaren Salze.

Besonders wichtig sind Verbindungen der Formel IB

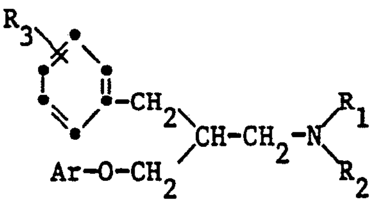

(IB),

worin $R_1$ und $R_2$ unabhängig voneinander Wasserstoff oder Niederalkyl bedeuten; $R_3$ für Wasserstoff, Halogen oder Trifluormethyl steht; Ar Phenyl bedeutet, welches durch einen oder zwei, gleiche oder verschiedene Substituenten der Gruppe Niederalkyl, Niederalkoxy, Niederalkenyloxy, Niederalkylmercapto, Niederalkylsulfinyl, Niederalkylsulfonyl, Halogen oder Trifluormethyl substituiert ist; wobei die mit "nieder" bezeichneten Gruppen höchstens 7 Kohlenstoffatome enthalten und ihre Säureadditionssalze, insbesondere ihre therapeutisch verwendbaren Salze.

Sehr bevorzugt sind Verbindungen der Formel IB, worin $R_1$ Wasserstoff oder Alkyl mit 1-4 Kohlenstoffatomen bedeutet; $R_2$ für Alkyl mit 1-4 Kohlenstoffatomen steht; $R_3$ Wasserstoff oder Chlor bedeutet; Ar für Phenyl steht, welches durch Alkyl, Alkoxy oder Alkylmercapto mit jeweils 1-4 Kohlenstoffatomen, Chlor oder Trifluormethyl monosubstituiert ist; und ihre Säureadditionssalze, insbesondere ihre therapeutisch verwendbaren Salze.

Weitere bevorzugte Verbindungen sind diejenigen der Formel IB, worin $R_1$ Wasserstoff ist, $R_2$ Alkyl mit 1-4 Kohlenstoffatomen bedeutet; $R_3$ für Wasserstoff oder Chlor steht; Ar Phenyl bedeutet, das durch Alkyl, Alkoxy oder Alkylmercapto mit jeweils 1-4 Kohlenstoffatomen, Chlor oder Trifluormethyl monosubstituiert ist, und ihre Säureadditionssalze, insbesondere ihre therapeutisch verwendbaren Salze.

In allen genannten Verbindungen der Formel IB Ar bedeutet vorzugsweise Phenyl, welches in 3- und/oder 4-Stellung des Phenylrings durch die oben genannten Substituenten di- oder monosubstituiert ist.

Als Beispiele für die vorher genannten bevorzugten Verbindungen seien diejenigen der Formel IB genannt, in welchen $R_1$ Wasserstoff oder Methyl bedeutet; $R_2$ für Methyl steht; $R_3$ Wasserstoff oder Chlor in 3- oder 4-Stellung bedeutet; Ar für Phenyl steht, das durch Methoxy, Methyl, Methylthio, Chlor oder Trifluormethyl monosubstituiert ist, und ihre Säureadditionssalze, insbesondere therapeutisch verwendbaren Salze. Bevorzugt sind weiter von diesen Verbindungen diejenigen, worin Ar Phenyl bedeutet, das in 3- oder 4-Stellung, vorzugsweise in 4-Stellung, durch Methoxy, Methylthio oder Trifluormethyl substituiert ist, und ihre Säureadditionssalze, insbesondere ihre therapeutisch verwendbaren Salze.

Eine besonders wertvolle Gruppe von Verbindungen ist diejenige der Formel I, worin $R_1$ Wasserstoff oder Methyl ist, $R_2$ für Methyl steht; $R_3$ Wasserstoff, Niederalkyl, Niederalkoxy, Halogen oder Trifluormethyl bedeutet; n für die ganze Zahl 1 oder 2 steht, X Sauerstoff bedeutet und Ar für unsubstituiertes Phenyl steht, und ihre Säureadditionssalze, insbesondere ihre therapeutisch verwendbaren Salze.

Die hier verwendeten allgemeinen Definitionen haben im Rahmen der vorliegenden Erfindung die folgende Bedeutung.

Der Ausdruck "nieder" definiert in den oben oder nachfolgend genannten organischen Resten oder Verbindungen solche mit höchstens 7, vorzugsweise 4, insbesondere 1 oder 2 Kohlenstoffatomen.

Eine Niederalkylgruppe enthält vorzugsweise 1-4 Kohlenstoffatome und bedeutet zum Beispiel Ethyl, Propyl, Butyl oder vorzugsweise Methyl.

Niederalkoxy ist vorzugsweise Ethoxy, Propoxy, Isopropoxy oder insbesondere Methoxy.

Niederalkylthio ist vorzugsweise Ethylthio, Propylthio oder vorzugsweise Methylthio.

Niederalkylsulfinyl ist insbesondere Ethylsulfinyl, Propylsulfinyl oder vorzugsweise Methylsulfinyl.

Niederalkylsulfonyl ist vorzugsweise Ethylsulfonyl, Propylsulfonyl oder insbesondere Methylsulfonyl.

Eine Alkenylgruppe, als solche oder wie beispielsweise in Alkenyloxy vorhanden, bedeutet geradkettiges oder verzweigtes Alkenyl, vorzugswiese mit bis zu 7 Kohlenstoffatomen, insbesondere bis und mit 4 Kohlenstoffatomen, z. B. 2-Propenyl (Allyl), 2-Butenyl, 2-Methyl-2-propenyl oder 2-Methyl-2-butenyl.

Ein Alkinylrest, als solcher oder wie beispielsweise in Alkinyloxy vorhanden, bedeutet geradkettiges oder verzweigtes Alkinyl, vorzugsweise mit bis zu 7 Kohlenstoffatomen, insbesondere bis und mit 4 Kohlenstoffatomen, z. B. 2-Propinyl (Propargyl), 2-Butinyl oder 2-Pentinyl.

Halogen ist vorzugsweise Chlor, kann aber ebenso Brom, lod oder Fluor sein.

Wenn $R_1$ und $R_2$ gemeinsam mit dem benachbarten Stickstoffatom, zu welchem sie in der Formel I gebunden sind, einen fünf- oder sechsgliedrigen gesättigten heterocyclischen Ring bilden, dieser kann kein oder ein weiteres Heteroatom aufweisen. Fünf- oder sechsgliedrige gesättigte heterocyclische Ringe ohne ein weiteres Heteroatom sind vorzugsweise Pyrrolidino oder Piperidino. Fünf- oder sechsgliedrige gesättigte heterocyclische Ringe, welche ein zusätzliches Heteroatom enthalten, umfassen solche Ringe, in welchen die zusätzliche Heterogruppe NH, N-Niederalkyl, O oder S ist. Solche repräsentative Ringe sind vorzugsweise

Pyrazolidino, Isooxazolidino und vorzugsweise Morpholino, Thiomorpholino, Piperazino oder N-Niederalkylpiperazino.

Die genannten Verbindungen der Formel I bilden Säureadditionssalze, die vorzugsweise solche von therapeutisch verwendbaren anorganischen oder organischen Säuren sind, wie beispielsweise von starken Mineralsäuren, zum Beispiel Halogenwasserstoffsäuren, z. B. Salzsäure oder Bromwasserstoffsäure; Schwefel-, Phosphor-, Salpeter- oder Perchlorsäure; aliphatischen oder aromatischen Carbonsäuren oder Sulfonsäuren, z. B. Ameisen-, Essig-, Propion-, Bernstein-, Glykol-, Milch-, Äpfel-, Wein-, Glucon-, Zitronen-, Malein-, Fumar-, Pyruvin-, Phenylessig-, Benzoe-, 4-Aminobenzoe-, Anthranil-, 4-Hydroxybenzoe-, Salicyl-, 4-Aminosalicyl-, Pamoe-, Nikotin-; Methansulfon-, Äthansulfon-, Hydroxyäthansulfon-, Benzolsulfon-, p-Toluolsulfon-, Naphthalinsulfon-, Sulfanil-, Cyclohexylsulfaminsäure; oder von Ascorbinsäure.

Säureadditionssalze, z. B. solche von anderen starken anorganischen oder organischen Säuren, z. B. Perchlorsäure oder Pikrinsäure, können auch bei der Isolierung, Reinigung und Charakterisierung der Verbindungen der Formel I verwendet werden.

Die Verbindungen der vorliegenden Erfindung, zeigen wertvolle pharmakologische Eigenschaften, z. B. insbesondere regulierende Wirkungen auf das zentrale Nervensystem, spezifisch solche Effekte, die auf antidepressive Wirkungen hinweisen. Diese kommen u.a. durch die selektive Hemmung der Serotonin-Aufnahme der Gehirn-Nervenzellen zustande. Die Verbindungen der Erfindung hemmen auch die neuronale Aufnahme des Norepinephrins (NE).

Die obengenannten Eigenschaften können durch in vitro oder in vivo Tierversuche, vorzugsweise an Säugetieren, wie Mäusen, Ratten, Meerschweinchen, Kaninchen oder Affen, als Testobjekte nachgewiesen werden. Die genannten Verbindungen können ihnen enteral oder parenteral, vorzugsweise oral, oder subkutan, intravenös oder intraperitoneal, z. B. durch Gelatine-Kapseln oder in Form von Stärke enthaltenden Suspensionen oder wässrigen Lösungen bzw. Suspensionen, verabreicht werden. Die verwendete Dosis kann in einem Bereich von ungefähr zwischen 0,1 und 100 mg/kg/Tag, vorzugsweise ungefähr 1 und 50 mg/kg/Tag, insbesondere ungefähr 5 und 30 mg/kg/Tag liegen.

Die Hemmung der Serotonin (5-HT)-Aufnahme, die auf die potentielle antidepressive Wirkung hinweist, wird in vitro, an Synaptosomen des Mittelhirns der Ratte wie folgt bestimmt:

Es werden Synaptosomen aus der Mittelhirn-Zone der Ratte präpariert und man bestimmt die $^3H$-5-HT-Aufnahme nach Baumann and Maitre, Naunyn-Schmiedeberg's Arch. Pharmacol. <u>300</u>, 31, (1977). Die Konzentration von 5-HT im Inkubationsmedium ist $2{,}5 \times 10^{-9}$M und die Inkubation dauert <u>10</u> Minuten. Es wird diejenige Konzentration einer erfindungsgemässen Verbindung, welche die Serotonin-Aufnahme um 50% hemmt, d.h. der $IC_{50}$-Wert, bestimmt.

Als Beispiele der erfindungsgemässen Verbindungen seien das N,N-Dimethyl-2-benzyl-3-(4-methylthiophenoxy)-propylamin-oxalat, das N,N-Dimethyl-2-benzyl-3-(4-trifluormethyl-phenoxy)-propylamin-oxalat und das N-Methyl-2-benzyl-3-(4-trifluormethyl-phenoxy)-propylamin-oxalat genannt, welche die Aufnahme von $^3H$-5-HT in die Synaptosome des Mittelhirns der Ratte mit $IC_{50}$-Werten von ungefähr $3 \times 10^{-8}$ M, $1{,}5 \times 10^{-8}$ M bzw. $1 \times 10^{-8}$ M hemmen.

Die Hemmung der Norepinephrin (NE)-Aufnahme wird in vitro an Synaptosomen des Mittelhirns der Ratte wie folgt bestimmt:

Es werden Synaptosomen aus der Mittelhirn-Zone der Ratte präpariert und man misst die $^3H$-NE-Aufnahme nach Baumann and Maitre, Naunyn-Schmiedeberg's Arch. Pharmacol. <u>300</u>, 31 (1977). Die Konzentration von NE im Inkubationsmedium ist $1 \times 10^{-8}$M und die Inkubation dauert 20 Minuten. Es wird diejenige Konzentration einer neuen Verbindung, welche die Norepinephrin-Aufnahme um 50 % hemmt, d.h. der $IC_{50}$-Wert, bestimmt.

Als Beispiele der erfindungsgemässen Verbindungen seien das N,N-Dimethyl-2-benzyl-3-(4-methylthio-phenoxy)-propylamin-oxalat, N,N-Dimethyl-2-benzyl-3-(4-trifluoromethyl-phenoxy)-propylamin-oxalat und das N-Methyl-2-benzyl-3-(4-trifluoromethyl-phenoxy)-propylamin-oxalat genannt, welche die Aufnahme von $^3H$-NE in die Synaptosome des Mittelhirns der Ratte mit $IC_{50}$-Werten von ungefähr $5 \times 10^{-7}$M, $3 \times 10^{-6}$ M bzw. $2 \times 10^{-6}$ M hemmen.

Die Hemmung der Serotonin (5 HT)-Aufnahme wird auch in Ratten, welche man mit Verbindungen der vorliegenden Anmeldung vorbehandelt hat, bestimmt.

Als Beispiele der erfindungsgemässen Verbindungen seien das N,N-Dimethyl-2-benzyl-3-(4-methylthio-phenoxy)-propylamin-oxalat und das N,N-Dimethyl-2-benzyl-3-(4-trifluoromethyl-phenoxy)-propylamin-oxalat genannt, welche bei einer oralen Dosis von 30 mg/kg eine 80 %ige bzw. 60 %ige Hemmung der $^3H$-5HT-Aufnahme zeigen. Das N-Methyl-2-benzyl-3-(4-triflouromethyl-phenoxy)-propylamin-oxalat ergibt eine ungefähr 50 %ige Hemmung des $^3H$-5HT-Aufnahme bei einer Dosis von 10 mg/kg p.o.

Die Hemmung der Serotonin-Aufnahme oder die Eigenschaft der Aktivierung des Serotonins durch die Verbindungen der Erfindung weist auf die antidepressive Wirkung hin. Diese wird durch die Umkehr der durch Reserpin hervorgerufenen Ptosis (d.h. Kontraktion des Augenlids) am Kaninchen bestimmt.

Man verabreicht den Kaninchen durch intravenöse Injektion 2 mg/kg Reserpin 90 Minuten vor der intravenösen Infusion einer 0,2 %igen Wirkstofflösung in die Ohrenvene, gemäss der Methode von J. Mizoule et al., [J. Phermecol., <u>8</u>, 3, 269-285, (1977)]. Das vollständige Öffnen des Augenlids wird als Kriterium für die Umkehr der durch Reserpin hervorgerufenen Ptosis betrachtet. Die Resultate werden durch die Angabe der Durchschnittsdosis der Testverbindung, welche das vollständige Öffnen des Augenlids des mit Reserpin behandelten Kaninchens hervorruft, ausgedrückt. Als Beispiele der erfindungsgemässen Verbindungen seien das N,N-Dimethyl-2-benzyl-3-(4-methylthio-phenoxy)-propylamin-oxalat, das N,N-Dimethyl-2-benzyl-3-(4-trifluormethyl-phenoxy)-propylemin-oxalat und das N-Methyl-2-benzyl-3-(4-trifluormethyl-phenoxy)-propylamin-oxalat genannt, welche die beschriebene Wirkung bei einer i.v. Dosis von ungefähr 7,7, 12,6 bzw. 12,9 mg/kg zeigen.

Die Verbindungen der Erfindung hemmen auch die Serotonin-Absonderung hervorrufende Wirkung des 3-Hydroxy-4-methyl-α-ethylphenethylamins (H 75/12) im Rattengehirn. Der Test wird im wesentlichen gemäss Carlsson, Corrodi, Fuxe und Hoekfelt, Europ. J. Pharmacol. 5, 357 (1969) durchgeführt.

Die zur Prüfung ausgewählte Verbindung wird oral 30 Minuten vor den H 75/12 (25 mg/kg s.c.) verabreicht und die Tiere werden 4 Stunden später geköpft. Die Gehirne werden gleich entnommen, der Serotonin-Gehalt wird wie oben beschrieben bestimmt und der $ED_{50}$-Wert berechnet. Die Wirksamkeit einer erfindungsgemässen Verbindung bei der Hemmung des Effektes von H 75/12, der die Serotonin-Absonderung fördert, ist ein indirektes Mass der Fähigkeit zur Hemmung der Serotonin-Aufnahme.

Als Beispiele für die Illustration der hemmenden Wirkung der erfindungsgemässen Verbindungen auf die Serotonin-Aufnahme seien die $ED_{50}$-Werte im obigen H 75/12-Test für das N,N-Dimethyl-2-(3-chlorbenzyl)-3-(4-methylthio-phenoxy)-propylamin-oxalat und für das N-Methyl-2-benzyl-3-(4-trifluormethyl-phenoxy)-propylamin-oxalat genannt, welche bei ungefähr 1,4 bzw. 1,8 mg/kg p.o. liegen. Die $ED_{50}$-Werte für das N,N-Dimethyl-2-benzyl-3-(4-methylthio-phenoxy)-propylamin-oxalat und für das N,N-Dimethyl-2-benzyl-3-(4-trifluormethyl-phenoxy)-propylamin-oxalat sind ungefähr 2,3 bzw. 4,5 mg/kg p.o.

Dementsprechend können die Verbindungen der vorliegenden Erfindung, allein oder in Kombination bei Säugern für die Behandlung von psychotropischen Erkrankungen, insbesondere Depression, eingesetzt werden. Die Verbindungen der Erfindung können auch für die Behandlung von anderen Störungen des zentralen Nervensystems, welche auf die Regulierung des Gehirnserotonins ansprechen, z. B. minimale Funktionsstörungen des Gehirns, übermässiger Appetit, Angst, Schizophrenie und Dementia, verwendet werden. Die neuen Verbindungen können auch als Analgetika und zur Herstellung von anderen wertvollen Produkten, speziell von pharmakologisch wirksamen Präparaten, eingesetzt werden.

Die Verbindungen der Formel I der vorliegenden Erfindung können nach an sich bekannten Methoden hergestellt werden, indem man

a) eine Verbindung der Formel II

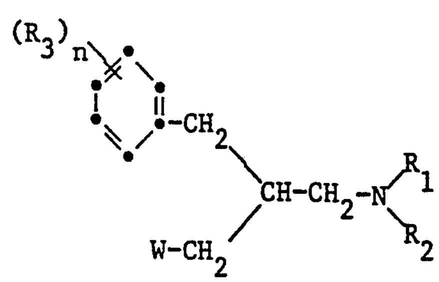

(II)

oder ein Säureadditionssalz davon, mit einer Verbindung der Formel III

Ar - Y (III),

worin $R_1$, $R_2$, $R_3$, n und Ar die oben angegebenen Bedeutungen haben, eines der Symbole W und Y die Gruppe -XH oder XH in der Form eines Metallsalzes bedeutet, worin X die obige Bedeutung hat, und das andere für die Gruppe XH in reaktionsfähig veresterter Form oder als ein N,N'-disubstituiertes Isoharnstoff- oder Isothioharnstoff-Derivat steht, kondensiert, oder

b) ein Amin der Formel IV

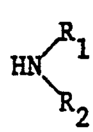

(IV)

oder ein Säureadditionssalz davon, mit einer Verbindung der Formel V

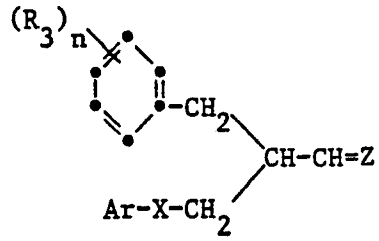

(V),

worin Z ein Wasserstoffatom zusammen mit einer reaktionsfähigen veresterten Hydroxygruppe oder die Oxogruppe bedeutet, und Ar, X $R_3$, und n die vorher angegebenen Bedeutungen haben, alkyliert, wobei wenn Z die Oxogruppe darstellt, wird die Alkylierung unter redukiven Bedingungen durchgeführt, oder

c) eine Verbindung der Struktur, die zu der oben definierten Verbindung der Formel I analog ist, und mindestens an einem mit dem Amino-Stickstoffatom benachbarten Kohlenstoffatom eine Oxogruppe aufweist, reduziert, oder

d) mehrfach Bindung(en) in einer Verbindung der Struktur, die zu der oben definierten Verbindung der Formel I analog ist, und worin im aliphatischen Teil des Moleküls, inklusive die Aminogruppe, eine oder zwei Doppelbindung(en) vorhanden sind, mit Wasserstoff sättigt, oder

e) in einer Verbindung der Formel VI

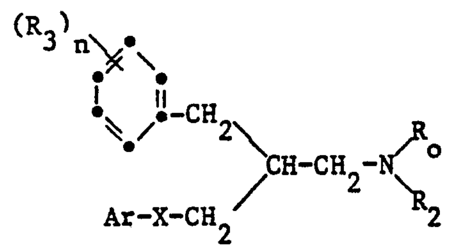

(VI),

worin $R_2$, $R_3$, X, n und Ar die oben angegebenen Bedeutungen haben, und $R_0$ eine Aminoschutzgruppe bedeutet, die Gruppe $R_0$ abspaltet, und eine Verbindung der Formel I, worin $R_1$ Wasserstoff ist, erhält, oder

f) eine Verbindung der Formel VII

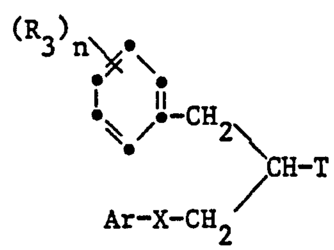

(VII),

worin T Cyan oder Azidomethyl bedeutet, $R_3$, n, X und Ar die obige Bedeutung haben, reduziert, und eine Verbindung der Formel I, worin jedes der Symbole $R_1$ und $R_2$ Wasserstoff bedeutet, erhält, oder

g) eine Verbindung der Formel VIIa

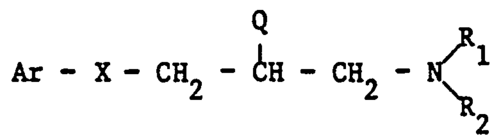

(VIIA)

mit einer Verbindung der Formel VIIB

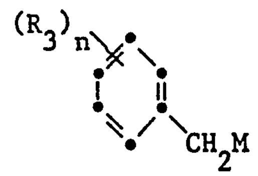

(VIIB),

worin eine der Gruppen M und Q eine abspaltbare Gruppe bedeutet und die andere ein Metall oder eine Halogenmetallgruppe ist, und Ar, X, $R_1$-$R_3$ und n die obige Bedeutung haben, unter vorübergehenden Schutz einer störenden Gruppe, kondensiert, und die erhaltene Verbindung der Formel I isoliert, und/oder, wenn erwünscht, eine erhaltene Verbindung der Formel I in eine andere Verbindung der Erfindung umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

Die Kondensation gemäss dem Verfahren a) wird wie unten beschrieben durchgeführt. Die Gruppe XH ist eine Hydroxy- oder Mercapto-(Thiol)-Gruppe. Die Metallsalz-Form einer solchen Gruppe ist vorzugsweise von einem Alkalimetall (z. B. Kalium, Lithium oder insbesondere Natrium) oder, bei der Mercaptogruppe, auch von ein- oder zweiwertigem Kupfer abgeleitet.

Die Kondensation gemäss dem Verfahren a) wird vorzugsweise unter Verwendung einer reaktionsfähigen funktionell abgewandelten Hydroxy- oder Thiolgruppe, welche vom Carbodiimid abgeleitet ist, durchgeführt. Diese Gruppe kann ein O-(oder S)-Derivat eines N,N'-disubstituierten Isoharnstoffs (oder Isothioharnstoffs) der charkateristischen Formel

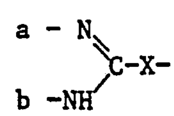

sein, worin X die obige Bedeutung hat und a und b gegebenenfalls substituierte Kohlenwasserstoffreste, z. B. insbesondere Cyclohexyl, bedeuten. Vorzugsweise werden die reaktionsfähigen Ester von diesem Typus in situ hergestellt und umgesetzt. Es wird z. B. ein Gemisch eines veresternden Reagenses, insbesondere ein N,N'-disubstituiertes Derivat des Carbodiimids, z. B. vorzugsweise N,N'-Dicyclohexylcarbodiimids und der Ausgangsstoffe II und III, worin W und Y in der Form einer freien Hydroxy bzw. Thiolgruppe ist, erhitzt. Im anderen Falle kann einer der Ausgangsstoffe, vorzugsweise bei einer Temperatur zwischen 50° und 200°C, mit dem N,N'-substituierten Carbodiimid, vorzugsweise in Gegenwart von Kupfer-I-chlorid, umgesetzt und das Isoharnstoff- oder Isothioharnstoffderivat hergestellt werden. Dann wird der zweite Ausgangsstoff hinzugefügt und zur Herstellung des Produktes das Gemisch weiter erhitzt.

Die Kondensation gemäss dem Verfahren a) kann auch nach einer weiteren bekannten Methode durchgeführt werden. Eine reaktionsfähige veresterte Form einer Gruppe XH ist vorzugsweise eine solche, worin die Hydroxy bzw. Thiolgruppe mit einer starken organischen Säure, z. B. einer aliphatischen oder aromatischen Sulfonsäure (z. B. einer Niederalkansulfonsäure, insbesondere Methansulfon-, Trifluormethansulfon- und Ethansulfonsäure, oder einer Arylsulfonsäure, insbesondere Benzolsulfon-, p-Toluolsulfon, p-Brombenzolsulfon- und p-Nitrobenzolsulfonsäure) oder mit einer starken anorganischen Säure, z. B., insbesondere Schwefelsäure oder eine Halogenwasserstoffsäure, z. B. Bromwasserstoff-, Jodwasserstoff- oder vorzugsweise Chlorwasserstoff-, oder falls sie an einen Ar-Rest angebunden ist, insbesondere mit Fluorwasserstoffsäure, verestert ist. Bedeutet das Symbol W oder Y eine mit einer Halogenwasserstoffsäure veresterte Gruppe XH, so steht es in der Tat für das entsprechende Halogenatom, nämlich für ein Brom-, Iod- oder vorzugsweise Chlor-, im Zusammenhang mit einem Rest Ar, für ein Fluoratom.

Bedeutet in einem Ausgangsstoff ArY, das Symbol Y ein Halogenatom, insbesondere Fluor, so ist günstig, wenn in ortho- oder para-Stellung eine aktivierende Gruppe, z. B. Trifluormethyl, Alkylsulfonyl oder

Alkylsulfinyl steht. Ein besonders günstiger Ausgangsstoff ist p-Fluorbenzotrifluorid (p-Trifluormethylfluorbenzol). Wird die Kondensation gemäss dem Verfahren a) wie oben beschrieben durchgeführt, so arbeitet man bei Temperaturen zwischen 0°C und dem Siedepunkt des Reaktionsgemische, vorzugsweise zwischen Raumtemperatur und 100°C. Vorzugsweise wird die Reaktion in Gegenwart eines gegenüber den Reagenzien inerten Lösungsmittels, z. B. in einem acyclischen oder cyclischen Ether (wie Diethylether, 1,2-Dimethoxyethan, Dioxan oder Tetrahydrofuran), und insbesondere eines tert.-Amids (welches ein nieders Molgewicht hat), z. B. N,N-Dimethylformamid, N-Methylpyrrolidon, N-Ethylpiperidon und Hexamethylphosphorsäure-triamid, durchgeführt. Wird eine starke Säure bei der Kondensation freigesetzt, so wird sie vorzugsweise durch Zugabe eines säurebindenden Mittels, z. B. eines Alkalimetallcarbonats oder -hydroxids, oder insbesondere einen sehr starken Base, z. B. eines Alkalimetallhydrids (z. B. Natrium- oder Kaliumhydrid), oder -alkoxids (z. B. Natriummethoxid oder -ethoxid, Kalium-tert.-butoxid) oder -amids (z. B. Lithium-diisopropylamid) gebunden. Ausgehend von einer Verbindung der Formel II, worin W Halogen (vorzugsweise Chlor) bedeutet, ist vorteilhaft, den entsprechenden Reaktionspartner der Formel III, in diesem Fall das Phenol, zuerst in das entsprechende Phenoxid, insbesondere Alkalimetallphenoxid (z. B. Natriumphenoxid) umzuwandeln. Man arbeitet dabei mit einer äquivalenten Menge Natriumhydrid in einem polaren aprotischen Lösungsmittel, z. B. in einem obengenannten, und führt die Kondensation mit diesem Salz durch. Bei der Kondensation der Ausgangsstoffe der Formeln II und III, in welcher die reaktionsfähige veresterte Hydroxygruppe in situ durch Einwirkung eines N,N'-disubstituierten Carbodiimids, z. B. N,N'-Dicyclohexylcarbodiimids, gebildet wird, arbeitet man unter an sich bekannten Bedingungen.

Die Ausgangsstoffe der Formel III sind allgemein bekannt, oder wenn neu, sie können leicht gemäss den für die bekannten Verbindungen beschriebenen Methoden hergestellt werden.

Die Ausgangsstoffe der Formel II sind im allgemeinen neu. Sie können nach konventionellen, an sich bekannten organischen Synthesen hergestellt werden. So kann man z. B. einen α-Benzylacrylsäureniederalkylester der Formel VIII

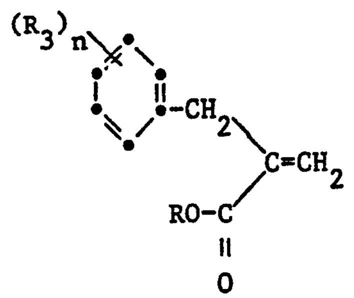

(VIII),

worin $R_3$ und n die oben angegebene Bedeutung haben und R Niederalkyl ist [für die Herstellung s. R.B. Miller und B.F. Smith, Synth. Commun. 3, 359 (1973)], mit einem oben definierten sekundären Amin der Formel IV kondensieren. Man arbeitet in einem Niederalkanol unter den Bedingungen der Michael-Addition, und den erhaltenen α-Benzyl-β-amino-propionsäure-niederalkylester der Formel

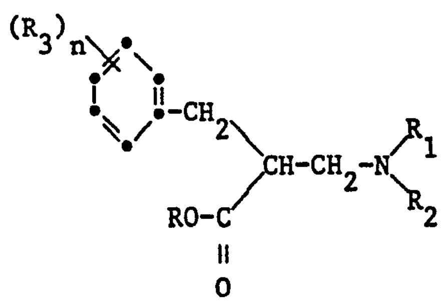

(IX),

worin $R_1$, $R_2$, $R_3$ und n die vorher amgegebene Bedeutung haben und Niederalkyl ist, reduziert man mit einem komplexen Hydrid, vorzugsweise Lithiumaluminiumhydrid oder einem äquivalenten Carbonyl-Reduktionsmittel, zu dem entsprechenden Alkohol, nämlich zu einer Verbindung der Formel II, worin W Hydroxy bedeutet. Wenn erwünscht, kann diese Hydroxygruppe mit konventionellen Mitteln in eine reaktionsfähige veresterte Form umgewandelt werden. Man verwendet z. B. für die Herstellung des bevorzugten Chlorids (W = Cl) die

Umsetzung mit Thionylchlorid, Phosphortrichlorid, Phosphoroxychlorid oder ein ähnliches Reagens.

Die Zwischenprodukte der Formel IX können auch durch a) Überführung z. B. eines Niederalkylesters der β-($NR_1R_2$)-substituierten Propionsäure in ein α-metalliertes Derivat, z. B. in das α-Lithio-Derivat mit Lithiumdiisopropylamid und b) nachfolgende Umsetzung des erhaltenen α-metallierten Derivates mit einer Verbindung der Formel IXA

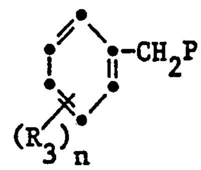

(IXA),

worin $R_3$ und n die oben angegebene Bedeutung haben und P eine vorher definierte reaktionsfähige veresterte Hydroxygruppe, insbesondere Halogen, z. B. Chlor bedeutet, erhalten werden. Man arbeitet in einem geeigneten Lösungsmittel, z. B. Tetrahydrofuran, gegebenenfalls in Gegenwart eines anderen Solvatisierungsmittels, z. B. Hexamethylphosphoramid.

Das Verfahren b) wird nach an sich bekannter Methode, die als Alkylierung von Aminen bezeichnet wird, durchgeführt. In den Ausgangsstoffen der Formel V kann die Gruppe Z (ersetzbar durch Amino) insbesondere ein Wasserstoffatom zusammen mit einer reaktionsfähigen veresterten Hydroxygruppe, oder die Oxogruppe bedeuten. Im ersten Fall erfolgt die Amin-Alkylierung unter den allgemeinen Bedingungen der substitutiven Alkylierung von Aminen, während im zweiten Fall die Bedingungen der reduktiven Alkylierung von Aminen angewendet werden.

Bei der substitutiven Alkylierung von Aminen der Formel IV ist die reaktionsfähige veresterte Hydroxygruppe in einer Verbindung der Formel V eine ähnliche wie diejenige im Verfahren a), insbesondere eine mit einer starken organischen Säure, z. B. einer aliphatischen oder aromatischen Sulfonsäure (z. B. einer Niederalkansulfonsäure, insbesondere Methansulfonsäure, Trifluormethansulfonsäure, Benzolsulfonsäure, p-Toluolsulfonsäure, p-Brombenzolsulfonsäure und p-Nitrobenzolsulfonsäure) oder mit einer starken anorganischen Säure, z. B. insbesondere Schwefelsäure, oder einer Halogenwasserstoffsäure, z. B. Chlorwasserstoff- oder, vorzugsweise lodwasserstoff- oder Bromwasserstoffsäure veresterte Hydroxygruppe. Die Alkylierung wird vorzugsweise unter ähnlichen Bedingungen und mit den gleichen Lösungsmitteln wie im Verfahren a) vorgenommen. Es sind jedoch auch halogenierte Alkane, z. B. Chloroform oder Methylenchlorid, nützliche Lösungsmittel. Üblicherweise werden auch mildere Säure-neutralisierende Mittel als im Verfahren a) verwendet, insbesondere organische quaternäre Basen (z. B. ein Tetrabutylammoniumsalz) oder organische tertiäre Basen, z. B. Triethylamin, N-Ethylpiperidin, Benzyldiethylamin, Pyridin oder Chinolin.

Die Kondensation gemäss dem Verfahren b) kann auch unter den Bedingungen der reduktiven Alkylierung von Aminen in an sich bekannten und verwendeter Weise, vorgenommen werden. Es wird eine Verbindung der Formel V, worin Z die Oxogruppe bedeutet und X, Ar, $R_3$ und n die obige Bedeutung haben, als Alkylierungsmittel verwendet. Dieses wird mit dem als Ausgangsstoff verwendeten Amin IV, und im gleichen oder nachfolgenden Reaktionsschritt mit einem Reduktionsmittel umgesetzt. Zwischen den Reduktionsmitteln, welche gleichzeitig mit dem Alkylierungsmittel verwendet werden, seien die komplexen Metallhydriden, wie Natriumcyanborhydrid genannt. Diejenigen Reduktionsmittel, welche hauptsächlich in einem separaten nachfolgenden Schritt eingesetzt werden seien das Diboran und komplexe Metallhydride, wie Natriumborhydrid und Natriumcyanborhydrid erwähnt, welche vorzugsweise im primären Reaktionsgemisch, ohne Isolierung des Zwischenproduktes, verwendet werden. In diesem Falle wird die Alkylierung vorzugsweise in einem gegenüber dem Reduktionsmittel inerten Lösungsmittel vorgenommen. Solche sind aliphatische oder cyclische Äther (z. B. Diethylether, Diisopropylether, 1,2-Dimethoxyethan, Dioxan oder Tetrahydrofuran) oder ein aliphatischer Alkohol (z. B. Methanol, Ethanol, Isopropylalkohol, Glykol, Glykolmonomethylether oder Diethylenglykol), welche vorzugsweise bei ungefähr 0°-80°C eingesetzt werden. Es ist jedoch das wichtigste Reduktionsmittel, welches sowohl simultan als auch nachfolgend verwendet werden kann, der Wasserstoff, insbesondere katalytisch aktivierter Wasserstoff. Die Katalysatoren sind die konventionell verwendeten Hydrierungskatalysatoren, vorzugsweise solche aus der Klasse von Edelmetallen (z. B. Palladium, Platin und Rhodium, auf einem Trägermaterial (z. B. Gasruss, Calciumcarbonat, Aluminiumoxid oder Bariumsulfat), in einer fein dispersierten Suspension ohne Trägermaterial, oder in Form von Komplexen, in einer homogenen Phase. Auch fein dispersierte Übergangsmetalle, z. B. Raney-Nickel, sind sehr geeignete Katalysatoren für die reduktive Alkylierung. Die spzeifischen Reaktionsbedingungen sind im hohen Masse vom betreffenden Hydrierungskatalysator und von seiner spezifischen Aktivität abhängig. Sie unterscheiden sich nicht von denjenigen, welche für die Hydrierung bekannt sind. Der Temperaturbereich zwischen Raumtemperatur und 150°C und ein Wasserstoffdruck zwischen atmosphärischem und ungefähr 300 Atmosphären kann gemäss den üblichen Standardmethoden angewendet werden. Ausser den oben im Zusammenhang mit der Hydridreduktion genannten inerten Lösungsmitteln können auch niedrigmolekulare Amide, insbesondere tertiäre Amide (z. B. N,N-Dimethylformamid, N,N-Dimethylacetamid, N-Methylpyrrolidon, N-Ethylpiperidon und Hexamethylphosphorsäure-triamid) sowie Formamid und Acetamid, als geeignete Lösungsmittel

eingesetzt werden. Spezielle Vorkehrungen sollen bei den Ausgangsstoffen der Formel V, worin X Schwefel bedeutet, getroffen werden. Da die meisten Hydrierungskatalysatoren durch Schwefelverbindungen inaktiviert werden, sollen andere Reduktionsmittel (z. B. die vorher genannten komplexen Metallhydride insbesondere Natriumcyanborhydrid) in diesem Falle bevorzugt werden.

Die als Ausgangsstoffe verwendeten Amine der Formel IV sind bekannte Verbindungen. Die neuen Ausgangsstoffe der Formel V können gemäss konventionellen synthetischen Methoden, erhalten werden. z. B. ausgehend von einer Verbindung der Formel X

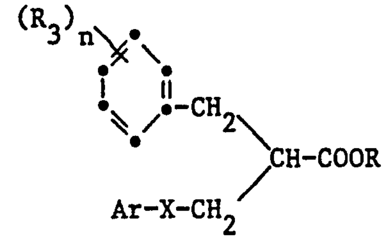 (X),

worin $R_3$, n, X und Ar die vorher angegebenen Bedeutungen haben, und R Wasserstoff oder Niederalkyl bedeutet, erhält man Verbindungen der Formel V, in welchen Z Oxo bedeutet oder Z für Wasserstoff zusammen mit einer reaktionsfähigen Hydroxygruppe steht.

Zwischenprodukte der Formel X können vorteilhafterweise durch Kondensation einer Verbindung der Formel VIII mit einer Verbindung der Formel Ar-XH, worin X für O, S oder N-Niederalkyl steht, oder ihren Alkalimetallsalzen, unter den allgemeinen Bedingungen der Michael-Addition, hergestellt werden. Die Verbindungen der Formel X können zu Verbindungen der Formel V, worin Z Wasserstoff zusammen mit einer Hydroxygruppe bedeutet, gemäss konventionellen Methoden reduziert werden. Man verwendet insbesondere komplexe Metallhydride unter Standardbedingungen. Die Reduktion von Verbindungen der Formel X mit z. B. Diisobutyl-aluminiumhydrid ergibt auch die Aldehyde der Formel V, worin Z Oxo bedeutet. Im anderen Falle kann man die Aldehyde auch durch Oxidation der obigen Verbindungen der Formel V, worin Z Wasserstoff zusammen mit einer Hydroxygruppe bedeutet, erhalten. Man verwendet z. B. Pyridinium-dichromat in Methylenchlorid. Sind Ausgangsstoffe erwünscht, in welchen Z Wasserstoff zusammen mit einer reaktionsfähigen veresterten Hydroxygruppe bedeutet, so wird die freie Hydroxygruppe in ihre reaktionsfähige veresterte Form gemäss konventionellen Verfahren, welche für die Veresterung mit starken Säuren eingesetzt werden, umgewandelt. So werden Halogenide durch Umsetzung mit üblichen Halogenierungsmitteln, welche vorher im Zusammenhang mit der Herstellung von analogen Ausgangsstoffen der Formel II genannt wurden, hergestellt.

Das Verfahren c) kann auch in an sich bekannter Weise, wie dies für die Reduktion von Amiden zu Aminen allgemein üblich ist, durchgeführt werden.

In den Ausgangsstoffen kann ein oder zwei dem Stickstoffatom benachbarte Kohlenstoffatome eine Oxogruppe tragen und eine Amid- oder Imidgruppe bilden. Die Ausgangsstoffe für dieses Verfahren können mit der Formel XI

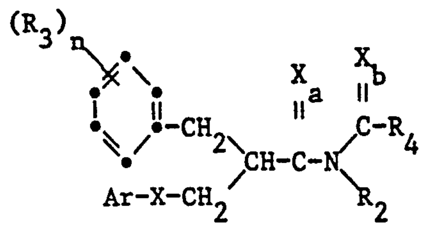 (XI)

dargestellt werden, worin X, Ar, $R_2$, $R_3$ und n die vorher angegebenen Bedeutungen haben, $R_4$ Wasserstoff oder ein Kohlenstoffatom weniger als $R_1$ enthaltendes Niederalkyl bedeutet; oder $R_2$ und $R_4$ zusammen einen fünf- oder sechsgliedrigen heterocyclischen Ring bilden; eines der Symbole $X_a$ und $X_b$ bedeutet Oxo und das andere für Oxo steht oder zwei Wasserstoffatome bedeutet. Die Reduktion wird mit einem konventionellen, geeigneten Reduktionsmittel, z. B. mit einem komplexen Metallhydrid, wie Lithiumaluminiumhydrid oder

Boran, durchgeführt.

Die Ausgangsstoffe der Formel XI sind neu und sie können gemäss an sich bekannten Verfahren hergestellt werden. So können Verbindungen der Formel XI, worin $X_a$ Oxo bedeutet und $X_b$ für zwei Wasserstoffatome steht durch einfache Amidierung von Zwischenprodukten der Formel IV, welche Verbindungen vorher definiert sind, hergestellt werden.

Ausgangsstoffe der Formel XI, worin X zwei Wasserstoffatome bedeutet und $X_b$ für Oxo steht, können z. B. durch Acylierung einer Verbindung der Formel I, worin mindestens einer der Reste $R_1$ und $R_2$ Wasserstoff bedeutet, erhalten werden. Man verwendet dabei ein reaktionsfähiges Derivat einer Alkansäure, z. B. Acetylchlorid.

Das Verfahren d) kann auch in an sich bekannter Weise, unter konventionellen Bedingungen, durchgeführt werden. Die Doppelbindung in den Ausgangsstoffen des Verfahrens d) kann entweder zwischen dem Stickstoffatom und einem benachbarten Kohlenstoffatom oder zwischen zwei Kohlenstoffatomen sein. So kann eine Kohlenstoffbindung zwischen dem Stickstoffatom und irgendeinem der benachbarten Kohlenstoffatome sein, während sich eine zusätzliche Kohlenstoff-Kohlenstoff Doppelbindung am zentralen Kohlenstoffatom befinden kann. Es können auch solche Ausgangsstoffe, in welchen nur eine Kohlenstoff-Kohlenstoff Doppelbindung vorhanden ist, verwendet werden. In diesen ist die Doppelbindung zwischen dem zentralen Kohlenstoffatom und irgendeinem der benachbarten Kohlenstoffatome. Die Ausgangsstoffe des Verfahrens d) können mit der Formel XIIa

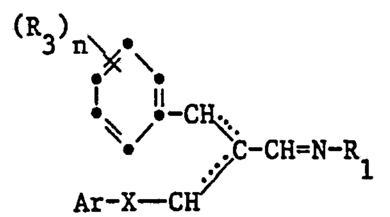

(XIIa),

worin $R_1$, $R_3$, n, Ar und X die oben angegebenen Bedeutungen haben, und die punktierten Linien eine gegebenenfalls vorhandene Doppelbindung bedeuten, oder mit der Formel XIIb

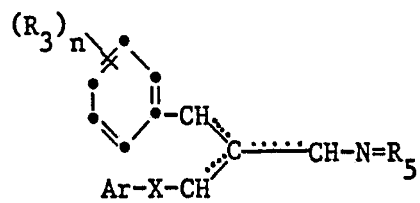

(XIIb),

worin $R_3$, n, Ar und X die vorher angegebene Bedeutung haben, $R_5$ einen Niederalkylidenrest bedeutet und die punktierten Linien eine gegebenenfalls vorhandene Doppelbindung bedeuten, oder mit der Formel XIII

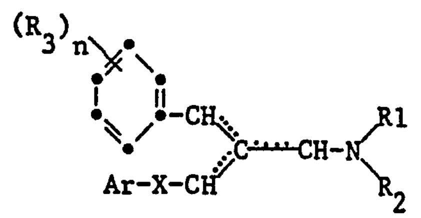

(XIII),

dargestellt werden, worin $R_1$, $R_2$, $R_3$, n, Ar und X die vorher angegebene Bedeutung haben und die punktierten Linien eine Doppelbindung bedeuten.

Das Verfahren e) kann in an sich bekannter Weise, insbesondere unter den Bedingungen der Abspaltung einer Aminoschutzgruppe durchgeführt werden. Geeignete Schutzgruppen, sowie Verfahren zu ihrer Einführung und Abspaltung sind aus der Literatur zur Genüge bekannt. Diese sind insbesondere als allgemeine Methoden für die Synthese von Peptiden in Einzelheiten entwickelt worden. Es sei z. B. auf Houben-Weyl: Methoden der organischen Chemie, 4. Ausgabe, Vol. 15/I und II, E. Wunsch (Herausgeber): Synthese von Peptiden (Georg Thieme Verlag, Stuttgart, 1974) hingewiesen.

Geeignete Amino-Schutzgruppen ($R_0$) sind insbesondere solche, welche durch Reduktion abgespalten werden können. Insbesondere sind als solche Gruppen z. B. die Benzyl- oder Benzyloxycarbonylgruppen, welche im aromatischen Teil durch Halogen, Niederalkoxy, Niederalkyl und vorzugsweise durch Nitrogruppen substituiert sein können, zu nennen. Im anderen Falle ist die Amino-Schutzgruppe Isonicotinyloxycarbonyl.

Die Benzyl-, Benzyloxycarbonyl- und Isonicotinyloxycarbonyl-Amino-Schutzgruppen können durch Hydrogenolyse, insbesondere über einem Edelmetallkatalysator, in üblicher Weise leicht abgespalten werden. Solche Gruppen, die Isonicotinylreste (z. B. Isonicotinyloxycarbonyl) enthalten und insbesondere substituierte Benzylreste, in erster Linie 4-Nitrobenzylreste jeglicher Art, können vorzugsweise durch Reduktion mit Zink, üblicherweise in Gegenwart einer Säure, vorzugsweise Essigsäure, und mit oder ohne Zusatz eines inerten Lösungsmittels, meistens bei Raumtemperatur, abgespalten werden.

Die Verwendung von $R_0$-Gruppen, die durch Acidolyse abspaltbar sind, ist auch möglich. Solche sind Cyan, tert.-Butyloxycarbonyl- und analoge Gruppen, aber auch solche vom Aralkyltypus wie Benzhydryl, Di-(4-methoxy)-benzhydryl und Triphenylmethyl (Trityl), oder gewisse Aralkoxycarbonylgruppen vom Typus des 2-(p-Biphenylyl)-2-propoxy-carbonyl-Typus, welche in der Schweizer Patentschrift 509 266 beschrieben sind. Die Abspaltung der Schutzgruppe durch Hydrolyse in saurem Medium (Acidolyse) wird bei den Gruppen vom tert.-Butyl-Typus mit Säuren wie Chlorwasserstoff-, Fluorwasserstoff- oder Trifluoressigsäure, und im Falle von Säure-empfindlichen Schutzgruppen hauptsächlich mit niederaliphatischen Carbonsäuren wie Ameisensäure und/oder Essigsäure, in Gegenwart von Wasser, und, gegebenenfalls eines polyhalogenierten Niederalkanols oder Niederalkanons, z. B. 1,1,1,3,3,3-Hexafluoropropan-2-ol oder Hexafluoroaceton, durchgeführt. In dieser Weise ist es möglich z. B. eine N-Tritylgruppe mit einer organischen Säure, z. B. Ameisensäure, Essigsäure, Chloressigsäure oder Trifluoressigsäure, in wässeriger Lösung oder in absolutem Trifluoräthanol als Lösungsmittel (vgl. Deutsche Offenlegungsschrift DT 2 346 147) oder mit wässeriger Essigsäure, abzuspalten.

Weiter kann z. B. die tert.-Butoxycarbonylgruppe mit Trifluoressigsäure oder Chlorwasserstoffsäure; die 2-(p-Biphenylyl)-isopropoxycarbonylgruppe mit wässeriger Essigsäure oder z. B. mit einem Gemisch von Eisessig-Ameisensäure (82,8 %)-Wasser (7:1:2) oder gemäss dem Verfahren von DT 2 346 147, abgespalten werden. Eine vorteilhafte Amino-Schutzgruppen $R_0$ ist auch die Ethoxycarbonylgruppe, welche in β-Stellung eine durch drei Kohlenwasserstoffreste substituierte Silylgruppe trägt, z. B. Triphenylsilyl, Dimethylbutylsilyl oder insbesondere Trimethylsilyl. Eine β-(Trihydrocarbyl-silyl)-ethoxycarbonylgruppe von diesem Typus, z. B. eine β-(Tri-niederalkyl-silyl)-ethoxycarbonylgruppe, z. B. insbesondere das β-(Trimethylsilyl)-äthoxycarbonyl bildet mit der zu schützenden Aminogruppe eine entsprechende β-Trihydrocarbylsilylethoxycarbonylaminogruppe (z. B. die β-Trimethylsilyl-ethoxycarbonylaminogruppe). Diese können vorzugsweise mit Reagenzien, welche Fluorid-Ione abgeben, z. B. mit Fluoriden von quaternären organischen Basen, z. B. Tetraethylammoniumfluorid, gespalten werden. Die Cyangruppe wird vorzugsweise durch Hydrolyse mit einer starken Mineralsäure, z. B. konzetrieter Chlorwasserstoffsäure, gemäss Chem. Ber. 68, 2291 (1935) abgespalten werden.

Die Ausgangsstoffe des Verfahrens e), nämlich die oben definierten Verbindungen der Formel VI, sind neu. Sie können nach einer bereits oben beschriebenen Methode, vorzugsweise Verfahren a), erhalten werden. Der einzige Unterschied ist, dass in den Ausgangsstoffen das Stickstoffatom der Gruppe-$NR_1R_2$ anstelle des Restes $R_1$ die Aminoschutzgruppe $R_0$ trägt.

Das Verfahren f) wird gemäss den bekannten Hydrierungsverfahren für die Reduktion von Nitrilen und Aziden, z. B. in Gegenwart von Ammoniak, oder durch Behandlung mit einfachen oder komplexen Metallhydriden, z. B. Alan, vorgenommen.

Die Ausgangsstoffe, z. B. Verbindungen in welchen T Cyan bedeutet, sind vorteilhafterweise zugänglich,

indem man ein α-Benzyl-acrylnitril der Formel XIV

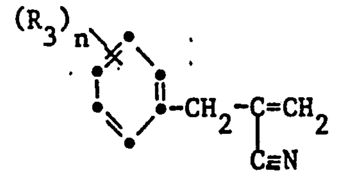

(XIV),

worin $R_3$ und n die obigen Bedeutungen haben, mit einer Verbindung der Formel ArXH, worin X O, S oder N-Niederalkyl bedeutet, oder z. B. ihre Alkalimetallderivate, unter den Bedingungen der Michael-Addition, kondensiert.

Das Verfahren g) kann nach an sich bekannten organometallischen Kupplungsverfahren durchgeführt werden. So kann man eine organometallische Verbindung VIIA oder VIIB, worin M oder Q ein Metallradikal, z. B. Aluminium, Magnesium, ein Alkalimetall, z. B. Lithium, Zink, Zirkonium oder Kupfer bedeutet, oder worin M oder Q für ein Halogenmetall-Radikal, z. B. Halogenmagnesium wie Chlormagnesium, steht, gegebenenfalls in Gegenwart eines Übergangsmetall-Katalysators, insbesondere eines Salzes davon, z. B. eines Halogenids von Chrom, Mangan, Eisen, Cobalt oder Nickel, z. B. wie in Comprehensive Organometallic Chemistry Vol. 2, 754; ibid. Vol. 8, 738 oder Tetrahedron 31, 2735 (1975) beschrieben, umsetzen. In den organometallischen Verbindungen VIIA und VIIB ist die Anzahl der zum Metall M oder Q gebundenen organischen Reste gleich der Wertigkeit des verwendeten Metalls.

Die Kondensation des organometallischen Reagenses mit einer Verbindung der Formel VIIA oder VIIB, worin M oder Q eine abspaltbare Gruppe, z. B. eine reaktionsfähige veresterte Hydroxygruppe [wie z. B. für das Verfahren a) beschrieben] bedeutet, wird vorzugsweise in einem inerten Lösungsmittel, z. B. in einem Ether wie Diethylether, Tetrahydrofuran oder Dimethoxyethan oder in einem Kohlenwasserstoff, z. B. Toluol vorgenommen. Man arbeitet vorzugsweise bei Temperaturen von ungefähr -75°C bis ungefähr +50°C, in einer inerten Atmosphäre und unter wasserfreien Bedingungen.

Das organometallische Reagens wird vorzugsweise in situ nach an sich bekannten Methoden hergestellt. So kann z. B. ein solches der Formel VIIB, worin M z. B. Lithium bedeutet, durch Behandlung eines gegebenenfalls geeignet substituierten Toluols mit z. B. Lithium-diisopropylamid, hergestellt werden. Die organometallische Verbindung der Formel VIIB, worin M Halomagnesium bedeutet (ein Grignard-Reagens) kann durch Umsetzung des gegebenenfalls substituierten Benzylhalogenids mit Magnesium, z. B. wie in Organic Synthesis Coll. Vol. I, 471 (1941) beschrieben, erhalten werden.

Die Isolierung des gewünschten Produkts der Formel I wird nach an sich bekannten Trennungsverfahren, z. B. fraktionierte Destillation oder Kristallisation, Dünnschichtchromatographie oder Hochdruck-Flüssigkeitschromatographie, durchgeführt.

Erfindungsgemässe Verbindungen, die nach irgendeinem der oben beschriebenen Verfahren erhalten werden, können nach konventionellen, dem Stand der Technik entsprechenden Methoden oder beispielsweise wie im folgenden illustriert, in eine andere umgewandelt werden.

Verbindungen der Formel I, worin $R_1$ und/oder $R_2$ Wasserstoff bedeutet, können in Verbindungen der Formel I, worin $R_1$ und/oder $R_2$ für Niederalkyl steht, umgewandelt werden, indem sie mit einem reaktionsfähigen Ester eines Niederalkanols, z. B. mit einem Niederalkylhalogenid umgesetzt werden. Man isoliert die erhaltene Verbindung der Formel I vorzugsweise als entsprechendes Säureadditionssalz. Man kann aber auch reduktiv alkylieren, z. B. mit Formaldehyd und Ameisensäure, wobei man Verbindungen der Formel I, worin $R_1$ und/oder $R_2$ Methyl bedeutet, erhält.

Verbindungen der Formel I, worin $R_1$ und $R_2$ Niederalkyl, vorzugsweise Methyl bedeutet, können in Verbindungen der Formel I, worin $R_1$ oder $R_2$ für Wasserstoff steht, durch katalytische Luft-Oxidation, z. B. mit Palladium-auf-Kohle, unter Verwendung eines Alkohols als Lösungsmittel, z. B. Methanol, vorzugsweise bei Raumtemperatur, umgewandelt werden. Man kann aber auch mit Halogenameisensäure-niederalkylester, z. B. Chlorameisensäure-ethylester umsetzen. Es werden die N-Acylderivate erhalten, welche zu den genannten unsubstituierten Verbindungen, solchem mit $R_1$ oder $R_2$ = H, z. B. mit einer Base, wie einem Alkalimetallhydroxid, z. B. mit einer Natriumhydroxidlösung in wässerigem Alkohol, hydrolysiert werden können.

Verbindungen der Formel I, worin $R_1$ und/oder $R_2$ Methyl bedeutet, können aus entsprechenden Verbindungen, in welchen $R_1$ und/oder $R_2$ für Wasserstoff steht, durch Umsetzung mit Halogenameisensäureniederalkylestern oder -phenylniederalkylestern, z. B. Halogenameisensäure-ethylester, in Verbindungen der Formel I, worin $R_1$ oder $R_2$ Alkoxycarbonyl oder Phenylalkoxycarbonyl ist, umgewandelt werden. Nach der Reduktion dieser Acylderivate mit einfachen oder komplexen Leichtmetallhydriden, z. B. Lithiumaluminiumhydrid, Natrium-tri-tert.butoxy-aluminiumhydrid oder Natrium-bis-(2-methoxyethoxy)-aluminiumhydrid, erhält man die Verbindungen, worin $R_1$ und/oder $R_2$ Methyl bedeutet.

Ungesättigte Verbindungen, z. B. solche, welche einen Alkenyl- oder Alkinylrest aufweisen, können mit katalytisch aktiviertem Wasserstoff in die Verbindungen der Formel I oder Zwischenprodukte, welche den entsprechenden Alkylrest haben, umgewandelt werden.

In allen obigen Reaktionen, kann es vorteilhaft sein, die potentiell reaktionsfähige, z. B. Amino-, Carboxy- oder andere störende Substituenten gemäss den an sich bekannten Schutzmethoden zu schützen. Es wird z. B. wie weiter unten illustriert, gearbeitet, um die störenden Reaktionen zu meiden. Man schützt solche Substituenten vor der gewünschten Reaktion und, wenn erwünscht, spaltet nachträglich die Schutzgruppen ab, wobei man die gewünschten Verbindungen, z. B. solche der Formel I oder Zwischenprodukte erhält.

So kann z. B. eine freie basische Aminogruppe, die Gruppe $-NR_1R_2$, die mindestens ein Wasserstoffatom am Stickstoffatom aufweist, in der Form von leicht spaltbaren Amiden, z. B. als Acylderivat wie Benzyloxycarbonyl(Carbobenzyloxy-)- oder als tert.-Butyloxycarbonyl-Derivat oder mit anderen leicht abspaltbaren N-Schutzgruppen, wie vorher beschrieben, geschützt werden.

In analoger Weise kann eine Carboxygruppe in der Form eines leicht spaltbaren Esters, z. B. Benzylester, Butylester, oder eines anderen allgemein verwendeten Esters, geschützt werden. In einer erhaltenen geschützten Verbindung der Formel I oder in einem Zwischenprodukt, worin eine oder mehrere funktionelle Gruppen geschützt sind, können die geschützten funktionellen Gruppen, z. B. Amino- oder Carboxygruppen, nach an sich bekannten Methoden, z. B. Solvolyse, insbesondere Hydrolyse mit einer Säure, oder durch Reduktion, insbesondere Hydrogenolyse, freigesetzt werden.

Die obengenannten Reaktionen werden nach an sich bekannten Methoden, in Gegenwart oder Abwesenheit von Verdünnungsmitteln, vorzugsweise solchen, welche gegenüber den Reagenzien inert sind und diese lösen, Katalysatoren, Kondensations- oder anderen oben genannten Mitteln, und/oder in einer inerten Atmosphäre, unter Kühlung, bei Zimmertemperatur oder bei erhöhter Temperatur, vorzugsweise beim Siedepunkt des verwendeten Lösungsmittels, bei normalem oder erhöhtem Druck, durchgeführt.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder optisch reinen Antipoden verwendet wird. Immer wenn gewünscht, werden die oben genannten Verfahren erst durchgeführt, nachdem alle potentiell störenden, reaktionsfähigen funktionellen Gruppen in geeigneter Weise geschützt sind, wie oben und in den Beispielen illustriert.

In den genannten Reaktionen werden vorteilhafterweise solche Ausgangsstoffe verwendet, welche zu den im vorstehenden als besonders bevorzugt beschriebenen Verbindungen führen.

Die Erfindung betrifft auch neue Ausgangsstoffe und Verfahren zu ihrer Herstellung.

Abhängig von den gewählten Ausgangsstoffen und Methoden können die neuen Verbindungen als eines der möglichen Isomeren oder als Isomerengemisch vorliegen, zum Beispiel als reine geometrische Isomeren (cis oder trans), als reine optische Isomeren wie etwa Antipoden oder als Mischungen aus optischen Isomeren wie etwa Racematen oder als Mischungen aus geometrischen Isomeren.

Erhaltene geometrische oder diastereomere Isomerengemische der obigen Verbindungen oder Zwischenprodukte können nach an sich bekannten Methoden in die einzelnen racemischen oder optisch aktiven Isomeren getrennt werden, z. B. durch fraktionierte Destillation, Kristallisation und/oder Chromatographie. Racemische Produkte können gleichfalls in die optischen Antipoden gespalten werden, z. B. durch Trennung ihrer diastereomeren Salze, wie gemäss J. Org. Chem. 43, 3803 (1978), z. B. durch fraktionierte Kristallisation von d- und λ-(Tartraten, Mandelaten, Camphersulfaten oder 1-Naphthyl-1-äthyliso-cyanaten) von Verbindungen, die eine basische, salzbildende Gruppe haben, oder von d- und λ-(α-Methylbenzylamin, Cinchonidin, Chinin, Chinidin, Ephedrin, Dehydroabietylamin, Brucin oder Strychnin)-Salzen von Verbindungen, die eine saure, salzbildende Gruppe besitzen.

Vorteilhafterweise wird jeweils das wirksamere Isomere und/oder der wirksamere Antipode der erfindungsgemässen Verbindungen isoliert.

Die Verbindungen der vorliegenden Erfindung werden schliesslich entweder in freier oder in Form ihrer Salze erhalten. Jede erhaltene Base kann in ein entsprechendes Säureadditionssalz umgewandelt werden, vorzugsweise unter Verwendung einer therapeutisch annehmbaren Säure oder eines Anionenaustauschers. Erhaltene Salze können in die entsprechenden freien Basen umgewandelt werden, zum Beispiel unter Verwendung einer stärkeren Base, beispielsweise eines Metall- oder Ammoniumhydroxids oder eines basischen Salzes, z. B. eines Alkalimetallhydroxids oder -carbonats, oder eines Kationenaustauschers. Diese oder andere Salze, etwa Pikrate, können auch zur Reinigung der erhaltenen Basen verwendet werden, indem man die Basen in Salze überführt, diese abtrennt und reinigt, und aus den Salzen wiederum die Base freisetzt. Infolge der engen Beziehung zwischen den freien Verbindungen und ihren Salzen sind in diesem Zusammenhang unter freien Verbindungen sinn- und zweckmässig gegebenenfalls immer auch die entsprechenden Salze zu verstehen.

Die Verbindungen und ihre Salze können auch in Form ihrer Hydrate erhalten werden, oder sie können andere zur Kristallisation verwendete Lösungsmittel einschliessen.

Die vorliegende Erfindung betrifft im weiteren Verbindungen der Formel I und ihre therapeutisch annehmbaren, nichttoxischen Säureadditionssalze zur Verwendung als Medikamente, und insbesondere zur

Verwendung bei der Herstellung pharmazeutischer Präparate, im besonderen Präparate, die eine hemmende Wirkung auf die Serotonin-Aufnahme, insbesondere antidepressive Wirkungen, haben.

Bei den erfindungsgemässen Präparaten handelt es sich um solche zur enteralen, wie oralen oder rektalen, sowie zur parenteralen Verabreichung an Säuger einschliesslich der Menschen. Die erfindungsgemässen pharmazeutischen Präparate enthalten mindestens eine Verbindung der Formel I, oder eines ihrer therapeutisch annehmbaren Salze als Wirkstoff, gegebenenfalls zusammen mit einem oder mehreren therapeutisch annehmbaren Trägerstoffen.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z. B. Lactose, Dextrose, Rohrzucker, Mannitol, Sorbitol, Cellulose und/oder Glycin, und Schmiermitteln, z. B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z. B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z. B. Stärken, Agar, Alginsäure oder ein Salz davon, wie Natriumalginat, und/oder Brausemischungen, oder Adsorptionsmittel, Farbstoffe, Geschmackstoffe und Süssmittel. Injizierbare Präparate sind vorzugsweise isotonische Lösungen oder Suspensionen, und Suppositorien in erster Linie Fettemulsionen oder -suspensionen. Die pharmakologischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch wertvolle Stoffe enthalten können, werden in an sich bekannter Weise, z. B. mittels konventioneller Misch-, Granulier- oder Dragierverfahren, hergestellt und enthalten von etwa 0,1 % bis etwa 75 %, insbesondere von etwa 1 % bis etwa 50 %, des Aktivstoffes.

Insbesondere betrifft die vorliegende Erfindung Methoden zur Behandlung psychotropischer Störungen, insbesondere Depression. Die Dosierung des Wirkstoffs hängt von der Warmblüterspezies, deren Körpergewicht, Alter und dem individuellen Zustand sowie der Applikationsweise ab. Einzeldosen für Säuger mit einem Gewicht von ungefähr 50-70 kg können zwischen ungefähr 10-100 mg des aktiven Bestandteils enthalten.

Die folgenden Beispiele dienen zur Illustration der Erfindung; sie sollen jedoch diese in ihrem Umfang in keiner Weise einschränken. Temperaturen werden in Celsiusgraden angegeben. Wenn nicht anders angegeben, werden alle Verdampfungs-Vorgänge bei vermindertem Druck, vorzugsweise zwischen ungefähr 15 und 100 mm Hg, durchgeführt.

**Beispiel 1:**

Eine Mischung aus 4.2 g 2-Benzyl-3-dimethylaminopropanol, 4.6 g Dicyclohexylcarbodiimid und 0.2 g Kupfer(I)chlorid wird 40 Stunden bei 80°C gehalten. Dann gibt man 5.0 g 4-Triflourmethylphenol hinzu und lässt die Mischung weitere 12 Stunden bei 130°C stehen. Das Reaktionsgemisch wird abgekühlt und 100 ml Ether hinzugegeben. Man filtriert die unlöslichen Bestandteile ab und extrahiert das Filtrat mit 2-n.Salzsäure (3x100 ml). Die sauren Extrakte werden vereinigt und durch Zugabe von konzentrierter wässeriger Ammoniaklösung basisch gemacht. Die wässerige Lösung wird dreimal mit je 150 ml Ether extrahiert, die vereinigten Extrakte über Magnesiumsulfat getrocknet und im Vacuum eingedampft, wobei man den gewünschten Ether als gelbes Öl erhält. Dieser wird in das Oxalat umgewandelt, indem man ihn in Ether löst und die berechnete Menge Oxalsäuredihydrat, gelöst in Ether, hinzugibt. Durch Filtration erhält man N,N-Dimethyl-2-benzyl-3-(4-trifluormethylphenoxy)propylamin-oxalat. F. 147-149°C.

Der Ausgangsstoff wird wie folgt hergestellt: Eine Lösung von 30.0 g α-Benzylacrylsäuremethylester, hergestellt nach der Methode von R.B. Miller und B.F. Smith [Synth. Commun. 3, 359 (1973)], in 50 ml Methanol wird zu 71.0 g Dimethylamin, gelöst in 210 ml Methanol, hinzugegeben und das Gemisch 48 Stunden bei Raumtemperatur gerührt. Das Lösungsmittel wird im Vacuum verdampft, und man erhält 2-Benzyl-3-dimethylaminopropionsäuremethylester als gelbes Öl, das ohne Reinigung weiterverwendet wird; NMR: δ 7.14 (s, 5H), 3.52 (s, 3H), 2.82 (m, 3H), 2.17 (s, 6H), 2.63-2.17 (m, übrige H).

Eine Lösung von 60.0 g 2-Benzyl-3-dimethylaminopropionsäuremethylester in 300 ml Ether wird in einer Stickstoffatmosphäre unter Rühren langsam zu einer Suspension von 28.0 g Lithiumaluminiumhydrid in 700 ml Ether getropft. Das Reaktionsgemisch wird 4 Stunden unter Rückfluss gekocht und dann 18 Stunden bei Raumtemperatur gerührt. Man kühlt das Gemisch auf 0°C ab und gibt vorsichtig, zunächst tropfenweise, 30 ml Wasser hinzu und dann 60 ml 10%ige wässerige Natronlauge. Die festen anorganischen Bestandteile werden abfiltriert und das Filtrat über Magnesiumsulfat getrocknet. Nach Verdampfen des Lösungsmittels im Vacuum erhält man 2-Benzyl-3-dimethylaminopropanol als Öl, das ohne weitere Reinigung verwendet wird; NMR ($CDCl_3$): δ 7.12 (m, 5H), 5.62 (breites s, 1H), 3.57 (m, 2H).

**Beispiel 2:**

Eine Mischung aus 5.0 g 2-(3-Chlorbenzyl)-3-dimethylamino-propanol, 3,3 g 4-Methylmercaptophenol und 4.5 g Dicyclohexylcarbodiimid wird 72 Stunden bei 180°C gehalten. Das Reaktionsgemisch wird auf Raumtemperatur abgekühlt und 100 ml Ether sowie 120 ml 1-n. Salzsäure hinzugefügt. Die wässerige Phase wird abgetrennt, mit weiteren 100 ml Ether gewaschen, dann durch Zugabe von konzentrierter wässeriger Ammoniaklösung basisch gemacht und mit 2 x 100 ml Ether extrahiert. Diese letzteren Ether-Extrakte werden vereinigt, über Kaliumcarbonat getrocknet und das Lösungsmittel im Vacuum verdampft. Durch Behandlung des erhaltenen Öls mit Oxalsäure, gelöst in Ether, erhält man N,N-Dimethyl-2-(3-chlorbenzyl)-3-(4-methylmercaptophenoxy)-propylamin-oxalat, F. 122-124°C.

**Beispiel 3:**

In analoger Weise erhält man gemäss den in den Beispielen 1 und 2 beschriebenen Methoden die folgenden Verbindungen der Formel 1, die in Form ihrer Oxalate isoliert und charakterisiert werden (Me = Methyl; Ph = Phenyl):

| Bsp. | $R_1$ | $R_2$ | $(R_3)_n$ | Ar | X | F.(°C) des Oxalats |
|---|---|---|---|---|---|---|
| 3/1 | Me | Me | H | 3-OMe-Ph | 0 | 85-88 (Zers.) |
| 3/2 | Me | Me | H | 4-SMe-Ph | 0 | 128-130 |
| 3/3 | Me | Me | H | 2-Cl-Ph | 0 | 91-93 (Zers.) |
| 3/4 | Me | Me | H | 4-Cl-Ph | 0 | 133-136 (Zers.) |
| 3/5 | $-(CH_2)_5-$ | | H | 4-OMe-Ph | 0 | 139-141 |
| 3/6 | Me | Me | H | 3-Cl-Ph | 0 | 112-114 |
| 3/7 | Me | Me | H | 4-Me-Ph | 0 | 115-117 |
| 3/8 | Me | Me | H | 3-Me-Ph | 0 | 94-97 |
| 3/9 | Me | Me | H | 4-OMe-Ph | S | 124-127 |
| 3/10 | Me | Me | H | $3-CF_3-Ph$ | 0 | 97-99 |
| 3/11 | Me | Me | H | 3-OMe-Ph | S | 94-97 |
| 3/12 | Me | Me | H | 3,5-di-OMe-Ph | 0 | 106-108 |
| 3/13 | Me | Me | H | $3-(OCH_2CH=CH_2)-Ph$ | 0 | 110-112 |
| 3/14 | Me | Me | 4-Cl | 4-SMe-Ph | 0 | 114-116 |
| 3/15 | Me | Me | 3-Cl | $4-CF_3-Ph$ | 0 | 138-140 |
| 3/16 | Me | Me | H | 4-OMe-Ph | 0 | 108-111 |

[(Zers.) = Zersetzung]

Die neuen Ausgangsstoffe werden wie folgt hergestellt:

a) α-(Chlorbenzyl)acrylsäureethylester werden folgendermassen hergestellt:

Eine Lösung von 26 g Kaliumhydroxid in 375 ml absolutem Ethanol wird innerhalb von 30 Minuten zu einer eisgekühlten Lösung von 112 g 3-Chlorbenzylmalonsäurediethylester in 375 ml absolutem Ethanol gegeben. Die Lösung wird 18 Stunden bei Raumtemperatur gerührt und dann das Lösungsmittel im Vacuum abgezogen. Der Rückstand wird in 1200 ml Wasser gelöst und die so entstandene Lösung in einem Eisbad gekühlt. Man gibt 29 ml konzentrierte Salzsäure hinzu und extrahiert die Lösung mit 2 x 350 ml Ether. Die vereinigten Etherextrakte werden mit 250 ml Wasser gewaschen, über Magnesiumsulfat getrocknet und im Vacuum eingedampft, wobei man 3-Chlorbenzylmalon-säuremonoethylester als Öl enthält; NMR ($CDCl_3$): δ 10.13 (s, 1H), 7.2 (m, 4H), 4.15 (q, 2H), 3.5 (m, 3H), 1.21 (t, 3H).

In einem Kolben, der mit einem Wasserbad gekühlt wird, werden 27.6 g (39 ml) Diethylamin tropfenweise unter Schütteln zu 90.0 g 3-Chlorbenzylmalonsäuremonoethylester [Chem. Ber. 92, 203 (1959)] gegeben. Unter Aufrechterhaltung der Kühlung werden 38 ml einer wässerigen Formaldehydlösung (37 %) ebenfalls vorsichtig unter Schütteln zugetropft. Man beobachtet eine Gasentwicklung, und die Farbe des Reaktionsgemisches wird heller. Die Mischung wird 65 Stunden bei Raumtemperatur gerührt, dann werden 500 ml Wasser hinzugegeben, worauf die Lösung mit 2 x 350 ml Ether extrahiert wird. Die vereinigten Etherextrakte werden zunächst mit 300 ml Wasser, dann 2 x mit je 200 ml 3-n. Salzsäure und nochmals mit 300 ml Wasser gewaschen. Die Etherlösung wird über Magnesiumsulfat getrocknet und das Lösungsmittel im Vacuum abgezogen, wobei man α-(3-Chlorbenzyl)acrylsäureethylester als gelbes Öl erhält. NMR ($CDCl_3$): δ 7.15 (m, 4H), 6.22 (s, 1H), 5.45 (m, 1H), 4.13 (q, 2H), 3.58 (s, 2H), 1.23 (t, 3H).

α-(4-Chlorbenzyl)acrylsäureethylester wird nach einem analogen Verfahren hergestellt, wobei man von 4-Chlorbenzylmalonsäurediethylester ausgeht (J.Med. Chem. 17, 732 (1974); NMR ($CDCl_3$): δ 7.15 (m, 4H), 6.17 (s,

1H), 5.38 (m, 1H), 4.11 (q, 2H), 3.55 (s, 2H), 1.20 (t, 3H).

b) Die folgenden Ester werden gemäss der im Beispiel 1 beschriebenen Methode bei den unten angegebenen Reaktionsbedingungen hergestellt:

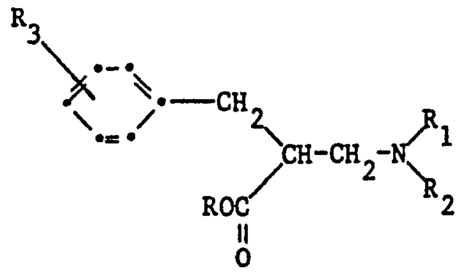

| $R_1$ | $R_2$ | $R_3$ | R | Reaktionsbedingungen |
|---|---|---|---|---|
| $-(CH_2)_5-$ | | H | Me | Methanol, Rückfluss, 18 Stunden |
| Et | Me | H | Me | Methanol, Rückfluss, 6 Stunden |
| $PhCH_2$ | Me | H | Me | Methanol, Rückfluss, 4 Wochen |
| Me | Me | 4-Cl | Et | Methanol, Raumtemperatur, 18 Stunden |
| Me | Me | 3-Cl | Et | Methanol, Raumtemperatur, 24 Stunden |
| H | Me | H | Me | Methanol, Raumtemperatur, 48 Stunden |

c) Die folgenden Alkohole werden gemäss der im Beispiel 1 beschriebenen Methode hergestellt:

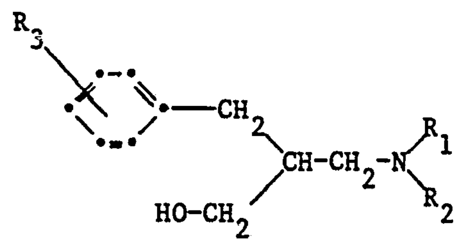

| $R_1$ | $R_2$ | $R_3$ | NMR (δ, für OH) |
|---|---|---|---|
| $-(CH_2)_5-$ | | H | 5.8 |
| Et | Me | H | 6.1 |
| $PhCH_2$ | Me | H | 5.0 |
| Me | Me | 4-Cl | 5.6 |
| Me | Me | 3-Cl | 5.6 |

**Beispiel 4:**

Eine Lösung aus 25.0 g N-Carbethoxy-N-methyl-2-benzyl-3-(4-trifluormethylphenoxy)propylamin und 25.0 g Kaliumhydroxid in 200 ml Isopropanol wird 48 Stunden unter Rückfluss gekocht. Das Reaktionsgemisch wird auf Raumtemperatur abgekühlt, und das Lösungsmittel im Vacuum verdampft. Man fügt 250 ml Wasser hinzu

und extrahiert die Mischung mit 2 x 150 ml Dichlormethan. Die vereinigten Dichlormethanextrakte werden mit 150 ml Wasser gewaschen, über Magnesiumsulfat getrocknet und das Lösungsmittel im Vacuum verdampf, wobei man ein Öl erhält. Dieses wird in 500 ml Ether gelöst, und man leitet solange getrockneten Chlorwasserstoff ein, bis kein weiterer Feststoff mehr ausfällt. Man filtriert den Feststoff ab, wäscht ihn mit 250 ml Ether, trocknet ihn und erhält N-Methyl-2-benzyl-3-(4-trifluormethylphenoxy)propylamin-hydrochlorid, F. 161-162°C, eine Verbindung der Formel I, in welcher $R_1$ Methyl, $R_2$ und $R_3$ Wasserstoff und Ar 4-Trifluormethylphenyl bedeuten. Das Oxalat, welches in gewohnter Weise hergestellt wird, hat einen F. von 173-175°C.

Der Ausgangsstoff wird wie folgt hergestellt:.

Eine Lösung von 22.0 g N,N-Dimethyl-2-benzyl-3-(4-trifluormethylphenoxy)propylamin und 34.1 g (30.0 ml) Chlorameisensäureethylester in 200 ml trockenem Toluol wird 72 Stunden unter Rückfluss gekocht. Das Reaktionsgemisch wird auf Raumtemperatur abgekühlt und das Lösungsmittel im Vacuum verdampft. 200 ml Wasser werden hinzugegeben, und die Mischung wird mit 2 x 150 ml Dichlormethan extrahiert. Die vereinigten Dichlormethanextrakte werden mit 200 ml 2-n.Salzsäure und 200 ml Wasser gewaschen und dann über Magnesiumsulfat getrocknet. Das Lösungsmittel wird im Vacuum verdampft, und man erhält N-Carbethoxy-N-methyl-2-benzyl-3-(4-triflourmethylphenoxy)propylamin als Öl, das ohne Reinigung weiterverwendet wird.

**Beispiel 5**:

Eine Lösung von 7.0 g N-Benzyl-N-methyl-2-benzyl-3-(3-methoxyphenoxy)propylamin in 100 ml Ethanol wird bei Raumtemperatur und bei atmosphärischen Druck bis zum Ende der Wasserstoffaufnahme hydriert, wobei als Katalysator 2.0 g 10 %-iger Palladium-auf-Kohle Katalysator verwendet werden. Der Katalysator wird abfiltriert und das Lösungsmittel im Vacuum verdampft, wobei man N-Methyl-2-benzyl-3-(3-methoxyphenoxy)propylamin als Öl erhält, das mit Oxalsäure, gelöst in Ether, zu N-Methyl-2-benzyl-3-(3-methoxyphenoxy)propylamin-oxalat, F. 127-129°C, umgesetzt wird.

Der Ausgangsstoff hat als Oxalat, das nach dem Verfahren des Beispiels 2 hergestellt wird, einen Schmelzpunkt von 79-81°C.

**Beispiel 6**:

Die folgenden Verbindungen der Formel I, in denen $R_2$ Wasserstoff und X Sauerstoff sind, und die als Oxalate isoliert und charakterisiert werden, werden in ähnlicher Weise wie die Verbindungen der Beispiele 4 und 5 analog den dort beschriebenen Verfahren hergestellt (Me = Methyl, Ph = Phenyl):

| Bsp. | $R_1$ | $(R_3)_n$ | Ar | F. (°C) des Oxalats |
|---|---|---|---|---|
| 6/1 | Me | H | 4-OMe-Ph | 142-144 |
| 6/2 | Me | H | 4-SMe-Ph | 172-174 |
| 6/3 | Me | 3-Cl | 4-SMe-Ph | 177-179 |

Ein Ausgangsstoff für das Beispiel 6/1 ist N,2-Dibenzyl-N-methyl-3-(4-methoxyphenoxy)propylamin-oxalat, F. 147-149°C.

**Beispiel 7**:

4.2 g einer 50 %-igen Natriumhydrid-Dispersion in Mineralöl wird 3 x mit je 100 ml Petrolether gewaschen und in einem trockenen Stickstoffstrom getrocknet. 65 ml Dimethylformamid, das mit Molekularsieb (4A) getrocknet ist, wird hinzugegeben. Weiterhin wird eine Lösung von 14 g 2-Benzyl-3-dimethylaminopropanol in 30 ml trockenem Dimethylformamid unter Rühren hinzugegeben. Das Rühren wird 10 Minuten bei Raumtemperatur fortgesetzt, und dann noch eine Stunde bei 55°C. Dann wird eine Lösung von 12.0 g 4-Fluorbenzotrifluorid in 25 ml trockenem Dimethylformamid vorsichtigt zugetropft, das Reaktionsgemisch wird 6 Stunden bei 60°C und dann noch 18 Stunden bei Raumtemperatur gerührt. Es werden 300 ml Wasser hinzugegeben, und die Lösung wird mit 2 x 200 ml Ether extrahiert. Die vereinigten Etherextrakte werden 2 x mit je 150 ml 3-n.Salzsäure extrahiert. Die vereinigten sauren Extrakte werden mit 200 ml Ether gewaschen und durch Zugabe von konzentrierter wässeriger Ammoniaklösung basisch gemacht. Die wässerige Lösung wird mit 2 x 250 ml Ether extrahiert, die vereinigten Etherextrakte mit 100 ml Wasser gewaschen und über Magnesiumsulfat getrocknet. Das Lösungsmittel wird im Vacuum verdampft, und man erhält N,N-Dimethyl-2-benzyl-3-(4-trifluormethylphenoxy)propylamin, das identisch ist mit der Verbindung aus Beispiel 1 und das zu

N,N-Dimethyl-2-benzyl-3-(4-trifluormethylphenoxy)propylamin-oxalat umgesetzt werden kann wie im Beispiel 1 beschrieben.

**Beispiel 8**:

Eine Mischung aus 2.0 g 10 %-igem Palladium-auf-Kohle Katalysator und N,N-Dimethyl-2-benzyl-3-(2-methoxyphenoxy)propylamin - eingesetzt werden 2 g des Oxalats, das bei 76 bis 79°C unter Zersetzung schmilzt - in 300 ml Methanol wird in einem offenen Kolben 18 Stunden bei Raumtemperatur gerührt. Der Katalysator wird abfiltriert und das Lösungsmittel im Vacuum verdampft. Das zurückbleibene Öl wird mit einer äquivalenten Menge Oxalsäure, gelöst in Ether, zu N-Methyl-2-benzyl-3-(2-methoxyphenoxy)propylamin-oxalat, F. 135-139° C, umgesetzt.

**Beispiel 9**:

Eine Lösung von 12.6 g N-Methyl-N-acetyl-2-benzyl-3-(4-methylthiophenoxy)propylamin in 100 ml trockenem Ether wird vorsichtig zu einer gerührten Suspension von 4.0 g Lithiumaluminiumhydrid in 250 ml trockenem Ether getropft. Das Reaktionsgemisch wird 18 Stunden unter Rückfluss gekocht und dann auf 0° C abgekühlt. Man gibt vorsichtig 3 ml Wasser hinzu, dann 3 ml 15 %-ige Natronlauge und nochmals 9 ml Wasser. Die anorganischen Salze werden abfiltriert, mit 150 ml Äther gewaschen und die vereinigten Etherextrakte über Magnesiumsulfat getrocknet. Das Lösungsmittel wird im Vacuum verdampft, und man erhält N-Methyl-N-ethyl-2-benzyl-3-(4-methylthiophenoxy)propylamin. F. 98-100° C.

Der Ausgangsstoff wird wie folgt hergestellt: Durch Umsetzung von N-Methyl-2-benzyl-3-(4-methylthiophenoxy)propylamin mit Acetylchlorid in Dichlormethan bei Raumtemperatur in Gegenwart von Triethylamin erhält man N-Methyl-N-acetyl-2-benzyl-3-(4-methylthiophenoxy)propylamin.

**Beispiel 10**:

Eine Lösung von 3.0 g N,N-Dimethyl-2-benzyl-3-(4-methylthiophenoxy)propylamin in 350 ml Methanol wird unter Stickstoff innerhalb von 30 Minuten zu einer Lösung von 4.8 g Natriumperjodat in 160 ml Wasser getropft. Nach erfolgter Zugabe wird das Reaktionsgemisch 4 Stunden bei Raumtemperatur gerührt, in 500 ml Wasser gegeben und mit 2 x 250 ml Ether extrahiert. Die vereinigten Etherextrakte werden über Kaliumcarbonat getrocknet und das Lösungsmittel im Vacuum verdampft, wobei man ein Öl erhält. Nach Umsetzung mit einer äquivalenten Menge an Oxalsäure, gelöst in Ether, und 3-maliger Kristallisation des entstandenen Salzes aus Methanol/Ether, erhält man N,N-Dimethyl-2-benzyl-3-(4-methylsulfinylphenoxy)-propylamin-oxalat, F. 112-115° C.

**Beispiel 11**:

Die folgenden Verbindungen der Formel I werden im wesentlichen gemäss den Verfahren der vorhergehenden Beispiele hergestellt:

| Verbindung | $NR_1R_2$ | X | $(R_3)_n$ | Ar | Salz | F°C |
|---|---|---|---|---|---|---|
| 11/1 | $N(CH_3)_2$ | O | H | $2\text{-}CH_3OC_6H_4$ | Oxalat | 76-79 Zers. |
| 11/2 | $N(CH_3)_2$ | O | H | $C_6H_5$ | Oxalat | 120-122 |
| 11/3 | $N(CH_3)_2$ | O | H | $3,4\text{-}CH_2O_2C_6H_3$ | Oxalat | 118-120 |
| 11/4 | $N(CH_3)_2$ | O | H | $3\text{-}CH_3SC_6H_4$ | Oxalat | 125-127 |
| 11/5 | $N(CH_3)C_2H_5$ | O | H | $3\text{-}CH_3OC_6H_4$ | Oxalat | 56-58 |
| 11/6 | $N(CH_3)_2$ | O | 3-Cl | $4\text{-}CH_3\text{-}\underset{\displaystyle O}{\underset{|}{S}}\text{-}C_6H_4$ | Oxalat | 106-108 |
| 11/7 | $NHCH_3$ | O | H | $C_6H_5$ | HCl | 136-138 |
| 11/8 | $N(CH_3)_2$ | $N\text{-}CH_3$ | H | $4\text{-}CH_3OC_6H_4$ | Oxalat | 118-120 |
| 11/9 | $N(C_2H_5)_2$ | O | H | $C_6H_5$ | HCl | 122-124 |

Die Ausgangsstoffe werden gemäss den vorhergehenden Beispielen hergestellt:

Der 2-Benzyl-3-dimethylamino-propionsäure-methylester (s. Beispiel 1) wird vorteilhafterweise auch wie folgt hergestellt:

Man kühlt 1800 ml Methanol auf -35° ab und versetzt es mit 500 g flüssigen Dimethylamin. Es wird dann eine Lösung von 500 g Acrylsäuremethylester in 850 ml Methanol hinzugefügt, wobei man die Temperatur zwischen -50° und -60° hält. Das Reaktionsgemisch wird 2 1/2 Stunden bei -50 bis -60° gehalten und dann über Nacht bei Raumtemperatur. Das Lösungsmittel wird im Vacuum abgedampft und der Rückstand wird unter vermindertem Druck destilliert. Man erhält den 3-Dimethylamino-propionsäure-methylester, der bei 60-70°/16 mm Hg siedet.

Eine Lösung von 101 g Diisopropylamin in 200 ml Tetrahydrofuran wird unter Stickstoff auf -78° gekühlt und bei dieser Temperatur tropfenweise mit 2,1 M n-Butyllithium in Tetrahydrofuran (470 ml) versetzt. Das Reaktionsgemisch wird bei dieser Temperatur 1/2 Stunde gerührt, mit einer Lösung von 179 g Hexamethylphosphoramid in 200 ml Tetrahydrofuran versetzt und bei -78° eine halbe Stunde weiter gerührt.

Bei der gleichen Temperatur wird es mit einer Lösung von 125 g N,N-Dimethylamino-propionsäure-methylester in 200 ml Tetrahydrofuran tropfenweise versetzt und 20 Minuten weiter gerührt. Bei -78° wird dann eine Lösung von 171 g Benzylbromid in 300 ml Tetrahydrofuran hinzugefügt. Die Lösung wird bei dieser Temperatur 2 Stunden gerührt, dann zuerst mit 250 ml gesättigter wässeriger Ammoniumchloridlösung und nachher mit 250 ml gesättigter wässeriger Natriumchloridlösung zersetzt. Das Gemisch wird mit 1500 ml Ether versetzt. Die Schichten werden getrennt und die wässerige Schicht wird mit 4 x 200 ml Ether extrahiert. Der Etherextrakt wird über Magnesiumsulfat getrocknet und zur Trockene eingedampft. Das erhaltene Öl wird wieder in 300 ml Ether gelöst, die Etherlösung mit 100 ml Wasser gewaschen und die wässerige Schicht mit 3 x 100 ml Ether wieder extrahiert. Die vereinigten Etherlösungen werden über Magnesiumsulfat getrocknet und zur Trockene eingedampft. Man erhält ein Öl, das durch fraktionierte Destillation gereinigt wird. Man erhält den 2-Benzyl-3-dimethylamino-propionsäure-methylester, der bei 104°/0,2-0,4 mm Hg siedet.

**Beispiel 12**:

Die Verbindungen der vorangegangenen Beispiele werden im Hinblick auf ihre Fähigkeit, die Aufnahme von tritium-markiertem Serotonin an den synaptosomalen Nervenenden beim Rattenganzhirn in vitro zu blockieren, getestet, wobei im wesentlichen gemäss der von Thornburg und Moore in Res. Comm. Chem. Pathol. Pharmacol. 5, 81 (1973) beschriebenen Methode verfahren wird. Die Präinkubations-Periode der Testverbindung (die in ethanolischer Lösung eingesetzt wird) mit der Grosshirn-Synaptosomen-Suspension in einer Krebs-Pufferlösung beträgt 5 Minuten. Die Inkubationszeit nach der Zugabe von tritium-markiertem Serotonin ($^3H$-5-HT) bis zu einer Endkonzentration von etwa 1 x $10^{-7}$ M beträgt 4 Minuten. Die Konzentration der Testverbindung, die nötig ist, um die Serotoninaufnahme um 50 % eines Kontrollwertes zu reduzieren, das heisst der $IC_{50}$-Wert, wird graphisch bestimmt und in mikromolarer Konzentration angegeben. Die Konzentration an Testverbindung, die benötigt wird, um die Norepinephrin (NE)-Aufnahme um 50 % eines Kontrollwertes zu reduzieren, das heisst der $IC_{50}$-Wert, wird ähnlich bestimmt, indem man tritium-markiertes Norepinephrin ($^3H$-NE) verwendet und den Wert ebenfalls in mikromolarer Konzentration ausdrückt.

| Verbindungen aus Beispiel | Hemmung der 5-HT-Aufnahme $IC_{50}$ (µM) | Hemmung der NE-Aufnahme $IC_{50}$ (µM) |
|---|---|---|
| 1 | 0.49 | 15.25 |
| 2 | 0.03 | 5.0 |
| 3/1 | 0.43 | 4.3 |
| 3/2 | 0.27 | 10.8 |
| 3/3 | 1.3 | 5.3 |
| 3/4 | 1.49 | 12.0 |
| 3/5 | 6.1 | 39.5 |
| 3/6 | 2.7 | 16.1 |
| 3/7 | 1.22 | 12.5 |
| 3/8 | 1.25 | 3.25 |
| 3/9 | 1.53 | 8.1 |
| 3/10 | 2.25 | 6.9 |
| 3/11 | 1.95 | 7.6 |
| 3/12 | 1.52 | 4.3 |
| 3/13 | 0.59 | 1.44 |
| 3/14 | 0.67 | 7.1 |
| 3/15 | 6.0 | 32.0 |
| 3/16 | 0.56 | 0.57 |
| 4 | 0.39 | 12.6 |
| 5 | 0.94 | 1.39 |
| 6/1 | 0.44 | 1.25 |
| 6/2 | 0.14 | 2.5 |
| 6/3 | 2.7 | 11.0 |
| 8 | 5.4 | 8.9 |

**Beispiel 13**:

Herstellung von 10'000 Tabletten mit einem Gehalt von je 10 mg der aktiven Substanz aus Beispiel 4:

**Bestandteile**:

| | |
|---|---|
| N-Methyl-2-benzyl-3-(4-trifluormethylphenoxy)-propylamin-hydrochlorid | 100.00 g |
| Milchzucker | 1157.00 g |
| Maisstärke | 75.00 g |
| Polyäthylenglykol 6000 | 75.00 g |
| Talkpulver | 75.00 g |
| Magnesiumstearat | 18.00 g |
| gereinigtes Wasser | q.s. |

**Verfahren**:

Sämtliche pulverigen Bestandteile werden mit einem Sieb von 0.6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 40 ml Wasser suspendiert und die Suspension zur siedenden Lösung von Polyethylenglykol in 150 ml Wasser gegeben. Die erhaltene Paste wird zu den Pulvern gegeben und gegebenenfalls unter Zugabe einer weiteren Wassermenge granuliert. Das Granulat wird über Nacht bei 35°C getrocknet, durch ein Sieb von 1.2 mm Maschenweite getrieben und zu Tabletten von 6.4 mm Durchmesser, welche eine Bruchrille aufweisen, gepresst.

**Beispiel 14**:

Herstellung von 10'000 Kapseln mit einem Gehalt von je 25 mg der aktiven Substanz aus Beispiel 4:

**Bestandteile**:

| | |
|---|---|
| N-Methyl-2-benzyl-3-(4-trifluormethylphenoxy)-propylamin-hydrochlorid | 250.0 g |
| Milchzucker | 1650.0 g |
| Kalkpulver | 100.0 g |

**Verfahren**:

Sämtliche pulverigen Bestandteile werden mit einem Sieb von 0.6 mm Maschenweite gesiebt. Dann wird der Wirkstoff zunächst mit Talk und darauffolgend mit dem Milchzucker in einem geeigneten Mischer homogenisiert. Kapseln Nr. 3 werden mit je 200 mg des Gemisches mit einer Füllmaschine gefüllt.
In analoger Weise werden Tabletten oder Kapseln, welche eine andere Verbindung der Erfindung, zum Beispiel eine solche der vorhergehenden Beispiele enthalten, hergestellt.

**Patentansprüche**

für die Vertragsstaaten BE, CH, DE, FR, GB, IT, LI, LU, NL, SE.

1. Verbindungen der allgemeinen Formel I

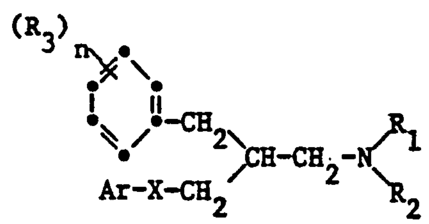

(I),

worin jedes der Symbole $R_1$ und $R_2$ Wasserstoff oder Niederalkyl bedeutet, oder $R_1$ und $R_2$ zusammen mit dem Stickstoffatom für Piperidino oder Pyrrolidino, oder für einen fünf- oder sechsgliedrigen gesättigten heterocyclischen Ring stehen, der NH, N-Niederalkyl, O oder S enthält; $R_3$ Wasserstoff, Niederalkyl, Niederalkoxy, Halogen oder Triflouromethyl bedeutet; Ar für gegebenenfalls durch einen bis drei Substituenten der Gruppe Niederalkyl, Niederalkoxy, Niederalkenyloxy, Niederalkinyloxy, Niederalkylmercapto, Niederalkylsulfinyl, Niederalkylsulfonyl, Halogen und Trifluormethyl, oder durch ein Niederalkylendioxy substituiertes Phenyl steht; X ein Sauerstoffatom, Schwefelatom oder N-Niederalkyl bedeutet, und n für die ganze Zahl 1, 2 oder 3 steht; ihre N-Oxide; ihre Säureadditionssalze, und ihre N-Niederalkyl oder N-Benzyl quaternären Salze, mit Ausnahme der Verbindung der Formel I, worin jedes der Symbole $R_1$ und $R_2$ Ethyl bedeutet, $R_3$ für Wasserstoff steht, Ar Phenyl bedeutet, X für ein Sauerstoffatom steht, und n 3 bedeutet, wobei die mit "nieder" bezeichneten Gruppen höchstens 7 Kohlenstoffatome enthalten, und ihrer Salze.

2. Verbindungen nach Anspruch 1, worin $R_1$ und $R_2$ unabhängig voneinander Wasserstoff oder Niederalkyl bedeuten, oder $R_1$ und $R_2$ zusammen mit dem Stickstoffatom Pyrrolidino oder Piperidino bedeuten oder für einen fünf- oder sechsgliedrigen gesättigten heterocyclischen Ring stehen, der NH, N-Niederalkyl, O oder S enthält, $R_3$ Wasserstoff Niederalkyl, Niederalkoxy, Halogen oder Triflourmethyl bedeutet, n für die ganze Zahl 1 oder 2 steht, X Sauerstoff, Schwefel oder N-Niederalkyl bedeutet, Ar für einen Phenylrest steht, der durch einen oder zwei Substituenten der Gruppe Niederalkyl, Niederalkoxy, Niederalkenyloxy, Niederalkinyloxy, Niederalkylmercapto, Niederalkylsulfinyl, Niederalkylsulfonyl, Halogen und Trifluormethyl, oder durch ein

Niederalkylendioxy substituiert ist, wobei die mit "nieder" bezeichneten Gruppen höchstens 7 Kohlenstoffatome enthalten, und ihre Säureadditionssalze.

3. Verbindungen der Formel IA

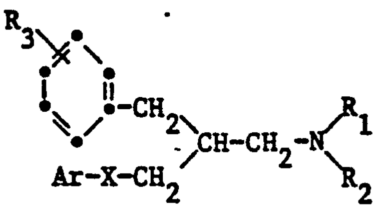

(IA),

worin $R_1$ und $R_2$ unabhängig voneinander Wasserstoff oder Niederalkyl bedeuten, oder $R_1$ und $R_2$ zusammen mit dem Stickstoffatom für Piperidino oder Pyrrolidino, oder für einen fünf- oder sechsgliedrigem gesättigten heterocyclischen Ring stehen, der NH, N-Niederalkyl, O oder S enthält; $R_3$ Wasserstoff, Niederalkyl, Niederalkoxy, Halogen oder Trifluormethyl bedeutet; Ar für einen Phenylrest steht, der durch einen oder zwei Substituentem der Gruppe Niederalkyl, Niederalkoxy, Niederalkenyloxy, Niederalkinyloxy, Halogen oder Trifluormethyl oder durch ein Niederalkylendioxy substituiert ist; und X für O oder S steht; wobei die mit "nieder" bezeichneten Gruppen höchstens 7 Kohlenstoffatome enthalten, und ihre Säureadditionssalze.

4. Verbindungen der Formel IA, worin $R_1$ und $R_1$ unabhänig voneinander Wasserstoff oder Niederalkyl mit 1 bis 7 Kohlenstoffatomen, bedeuten, oder $R_1$ und $_2$ zusammen mit dem Stickstoffatom für Pyrrolidino oder Piperidino stehen; $R_3$, Ar und X die im Anspruch 3 angegebenen Bedeutungen haben; und ihre Säureadditionssalze.

5. Verbindungen der Formel IB,

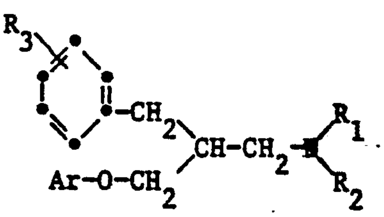

(IB),

worin $R_1$ und $R_2$ unabhängig voneinander Wasserstoff oder Niederalkyl bedeuten; $R_3$ für Wasserstoff, Halogen oder Trifluormethyl steht; Ar Phenyl bedeutet, welches durch einem oder zwei, gleiche oder verschiedene Substituenten der Gruppe Niederalkyl, Niederalkoxy, Niederalkenyloxy, Niederalkylmercapto, Niederalkylsulfinyl, Niederalkylsulfonyl, Halogen oder Trifluormethyl substituiert ist; wobei die mit "nieder" bezeichneten Gruppen höchstens 7 Kohlenstoffatome enthalten, und ihre Säureadditionssalze.

6. Verbindungen der im Anspruch 1 gezeigten Formel I, worin $R_1$ Wasserstoff oder Methyl ist, $R_2$ für Methyl steht; $R_3$ Wasserstoff, Niederalkyl, Niederalkoxy, Halogen oder Trifluormethyl bedeutet; n für die ganze Zahl 1 oder 2 steht, X Sauerstoff bedeutet und Ar für unsubstituiertes Phenyl steht, wobei die mit "nieder" bezeichneten Gruppen höchstens 7 Kohlenstoffatome enthalten, und ihre Säureadditionssalze.

7. N,N-Dimethyl-2-benzyl-3-(4-trifluormethylphenoxy)propylamin und seine Säureadditionssalze.

8. N,N-Dimethyl-2-(3-chlorbenzyl)-3-(4-methylmercaptophenoxy)propylamin und seine Säureadditionssalze.

9. N-Methyl-2-benzyl-3-(4-trifluormethylphenoxy)propylamin und seine Säureadditionssalze.

10. Pharmazeutische Präparate enthaltend Verbindungen der Ansprüche 1, 2 und 6, zusammen mit einem pharmazeutischen Trägermaterial.

11. Pharmazeutische Präparate enthaltend Verbindungen der Ansprüche 3, 4, 5 und 7-9, zusammen mit einem pharmazeutischen Trägermaterial.

12. Die in den Ansprüchen 1, 2 und 6 genannten Verbindungen zur Anwendung in einem Verfahren zur therapeutischen Behandlung des menschlichen oder tierischen Körpers.

13. Die in den Ansprüchen 3, 4, 5 und 7-9 genannten Verbindungen zur Anwendung in einem Verfahren zur therapeutischen Behandlung des menschlichen oder tierischen Körpers.

14. Die in den Ansprüchen 1, 2 und 6 genannten Verbindungen zur Verwendung als Antidepressiva.

15. Die in den Ansprüchen 3, 4, 5 und 7 bis 9 genannten Verbindungen zur Verwendung als Antidepressiva.

16. Verwendung der in den Ansprüchen 1, 2 und 6 genannten Verbindungen zur Herstellung von pharmazeutischen Präparaten.

17. Verwendung der in den Ansprüchen 3, 4, 5 und 7 bis 9 genannten Verbindungen zur Herstellung von pharmazeutischen Präparaten.

18. Verfahren zur Herstellung von den im Anspruch 1 genannten Verbindungen der Formel I, ihren N-Oxiden, Säureadditionssalzen und N-Niederalkyl- und N-Benzyl-quaternären Salzen, dadurch gekennzeichnet, dass man

a) eine Verbindung der Formel II

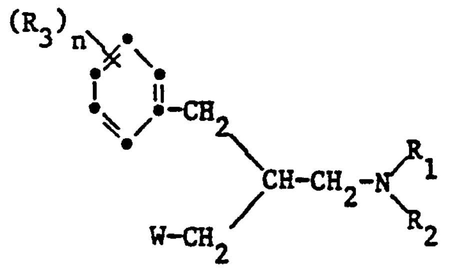

(II)

oder ein Säureadditionssalz davon, mit einer Verbindung der Formel III

Ar - Y (III),

worin $R_1$, $R_2$, $R_3$, n und Ar die oben angegebenen Bedeutungen haben, eines der Symbole W und Y die Gruppe -XH oder XH in der Form eines Metallsalzes bedeutet, worin X die obige Bedeutung hat, und das andere für die Gruppe XH in reaktionsfähig veresterter Form oder als ein N,N'-disubstituiertes Isoharnstoff- oder Isothioharnstoff-Derivat steht, kondensiert, oder

b) ein Amin der Formel IV

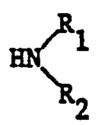

(IV)

oder ein Säureadditionssalz davon, mit einer Verbindung der Formel V

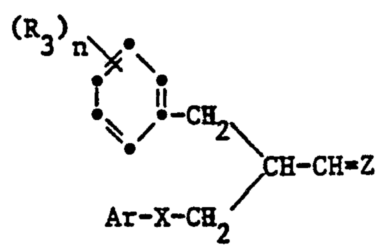

(V),

worin Z ein Wasserstoffatom zusammen mit einer reaktionsfähigen veresterten Hydroxygruppe oder die Oxogruppe bedeutet, und Ar, X, $R_3$ und n die vorher angegebenen Bedeutungen haben, alkyliert, wobei wenn Z die Oxogruppe darstellt, wird die Alkylierung unter reduktiven Bedingungen durchgeführt, oder

c) eine Verbindung der Struktur, die zu der oben definierten Verbindung der Formel I analog ist, und mindestens an einem mit dem Amino-Stickstoffatom benachbarten Kohlenstoffatom eine Oxogruppe aufweist, reduziert, oder

d) mehrfach Bindung(en) in einer Verbindung der Struktur, die zu der oben definierten Verbindung der Formel I analog ist, und worin im aliphatischen Teil des Moleküls, inklusive die Aminogruppe, eine oder zwei Doppelbindung(en) vorhanden sind, mit Wasserstoff sättigt oder

e) in einer Verbindung der Formel VI

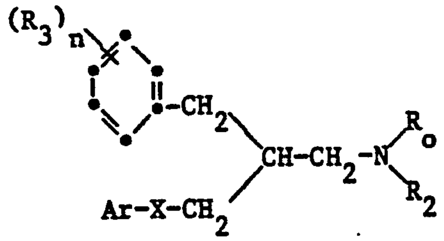

(VI),

worin $R_2$, $R_3$, X, n und Ar die oben angegebenen Bedeutungen haben, und $R_0$ eine Aminoschutzgruppe bedeutet, die Gruppe $R_0$ abspaltet, und eine Verbindung der Formel I, worin $R_1$ Wasserstoff ist, erhält, oder

f) eine Verbindung der Formel VII

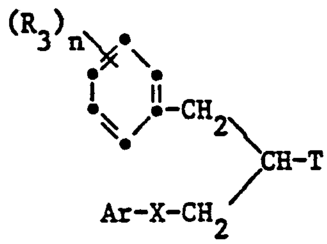

(VII),

worin T Cyan oder Azidomethyl bedeutet, $R_3$, n, X und Ar die obige Bedeutung haben, reduziert, und eine Verbindung der Formel I, worin jedes der Symbole $R_1$ und $R_2$ Wasserstoff bedeutet, erhält, oder

g) eine Verbindung der Formel VIIA

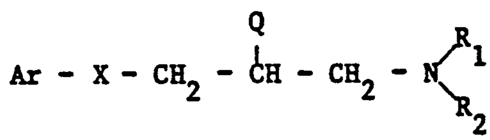

(VIIA)

mit einer Verbindung der Formel VIIB

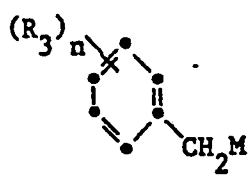

(VIIB),

worin eine der Gruppen M und Q eine abspaltbare Gruppe bedeutet und die andere ein Metall oder eine Halogenmetallgruppe ist, und Ar, X, $R_1$-$R_3$ und n die obige Bedeutung haben, kondensiert, wenn nötig, in allen diesen Verfahren eine störende Gruppe vorübergehend schützt, und die erhaltene Verbindung der Formel I

isoliert, und/oder, wenn erwünscht, eine erhaltene Verbindung der Formel I in eine andere Verbindung der Formel I umwandelt, und/oder, wenn erwünscht, eine erhaltene freie Verbindung in ein Salz oder ein erhaltenes Salz in die freie Verbindung oder in ein anderes Salz überführt, und/oder, wenn erhünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

**Patentansprüche**

für den Vertragsstaat AT.

1. Verfahren zur Herstellung von neuen Propylamin-Derivaten der allgemeinen Formel I

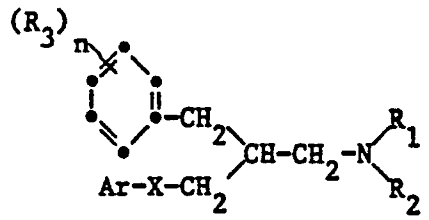

(I),

worin jedes der Symbole $R_1$ und $R_2$ Wasserstoff oder Niederalkyl bedeutet, oder $R_1$ und $R_2$ zusammen mit dem Stickstoffatom für Piperidino oder Pyrrolidino, oder für einen fünf- oder sechsgliedrigen gesättigten heterocyclischen Ring stehen, der NH, N-Niederalkyl, O oder S enthält; $R_3$ Wasserstoff, Niederalkyl, Niederalkoxy, Halogen oder Trifluormethyl bedeutet; Ar für gegebenenfalls durch einen bis drei Substituenten der Gruppe Niederalkyl, Niederalkoxy, Niederalkenyloxy, Niederalkinyloxy, Niederalkylmercapto, Niederalkylsulfinyl, Niederalkylsulfonyl, Halogen und Trifluormethyl, oder durch ein Niederalkylendioxy substituiertes Phenyl steht; X ein Sauerstoffatom, Schwefelatom oder N-Niederalkyl bedeutet, und n für die ganze Zahl 1, 2 oder 3 steht; ihren N-Oxiden, ihren Säureadditionssalzen und ihren N-Niederalkyl oder N-Benzyl quaternären Salzen, mit Ausnahme der Verbindung der Formel I, worin jedes der Symbole $R_1$ und $R_2$ Ethyl bedeutet, $R_3$ für Wasserstoff steht, Ar Phenyl bedeutet, X für ein Sauerstoffatom steht, und n 3 bedeutet, und ihrer Salze, wobei die mit "nieder" bezeichneten Gruppen höchstens 7 Kohlenstoffatome enthalten, dadurch gekennzeichnet, dass man

a) eine Verbindung der Formel II

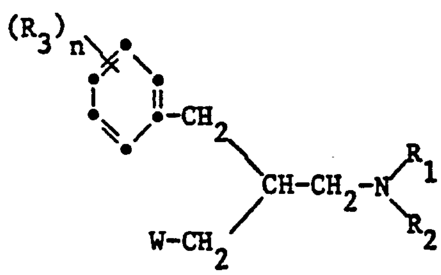

(II)

oder ein Säureadditionssalz davon, mit einer Verbindung der Formel III

Ar - Y (III),

worin $R_1$, $R_2$, $R_3$, n und Ar die oben angegebenen Bedeutungen haben, eines der Symbole W und Y die Gruppe -XH oder XH in der Form eines Metallsalzes bedeutet, worin X die obige Bedeutung hat, und das andere für die Gruppe XH in reaktionsfähig veresterter Form oder als ein N,N'-disubstituiertes Isoharnstoff- oder Isothioharnstoff-Derivat steht, kondensiert, oder

b) ein Amin der Formel IV

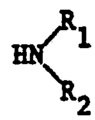

(IV)

oder ein Säureadditionssalz davon, mit einer Verbindung der Formel V

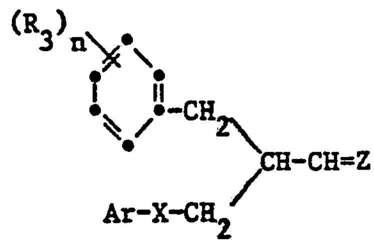

(V),

worin Z ein Wasserstoffatom zusammen mit einer reaktionsfähigen veresterten Hydroxygruppe oder die Oxogruppe bedeutet, und Ar, X, $R_3$ und n die vorher angegebenen Bedeutungen haben, alkyliert, und wenn die Gruppe Z Oxo bedeutet wird die Alkylierung unter reduktiven Bedingungen durchgeführt, oder

c) eine Verbindung der Struktur, die zu der oben definierten Verbindung der Formel I analog ist, und mindestens an einem mit dem Amino-Stickstoffatom benachbarten Kohlenstoffatom eine Oxogruppe aufweist, reduziert, oder

d) mehrfach Bindung(en) in einer Verbindung der Struktur, die zu der oben definierten Verbindung der Formel I analog ist, und worin im aliphatischen Teil des Moleküls, inklusive die Aminogruppe, eine oder zwei Doppelbindung(en) vorhanden sind, mit Wasserstoff sättigt, oder

e) in einer Verbindung der Formel VI

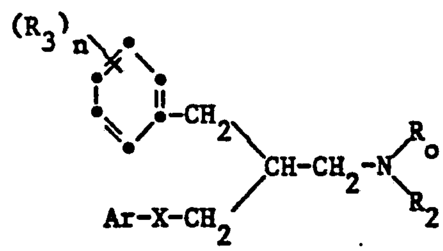

(VI),

worin $R_2$, $R_3$, X, n und Ar die oben angegebenen Bedeutungen haben, und $R_0$ eine Aminoschutzgruppe bedeutet, die Gruppe $R_0$ abspaltet, und eine Verbindung der Formel I, worin $R_1$ Wasserstoff ist, erhält, oder

f) eine Verbindung der Formel VII

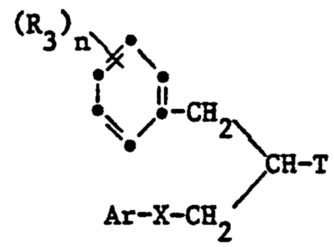 (VII),

worin T Cyan oder Azidomethyl bedeutet, $R_3$, n, X und Ar die obige Bedeutung haben, reduziert, und eine Verbindung der Formel I, worin jedes der Symbole $R_1$ und $R_2$ Wasserstoff bedeutet, erhält, oder

g) eine Verbindung der Formel VIIa

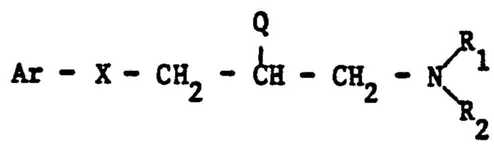 (VIIA)

mit einer Verbindung der Formel VIIB

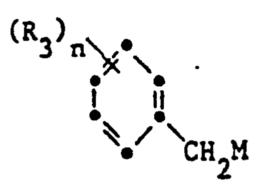 (VIIB),

worin eine der Gruppen M und Q eine abspaltbare Gruppe bedeutet und die andere ein Metall oder eine Halogenmetallgruppe ist, und Ar, X, $R_1$-$R_3$ undn die obige Bedeutung haben, kondensiert, wenn nötig, in allen diesen Verfahren eine störende Gruppe vorübergehend schützt, und die erhaltene Verbindung der Formel I isoliert, und/oder, wenn erwünscht, eine erhaltene Verbindung der Formel I in eine andere Verbindung der Formel I umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

2. Verfahren nach Anspruch 1, dadurch gekennzeichnet, dass man Verbindungen der Formel I, worin $R_1$ und $R_2$ unabhängig voneinander Wasserstoff oder Niederalkyl bedeuten, oder $R_1$ und $R_2$ zusammen mit dem Stickstoffatom Pyrrolidino oder Piperidino bedeuten oder für einen fünf- oder sechsgliedrigen gesättigten heterocyclischen Ring stehen, der NH, N-Niederalkyl, O oder S enthält, $R_3$ Wasserstoff, Niederalkyl, Niederalkoxy, Halogen oder Trifluormethyl bedeutet, n für die ganze Zahl 1 oder 2 steht, X Sauerstoff, Schwefel oder N-Niederalkyl bedeutet, Ar für einen Phenylrest steht, der durch einen oder zwei Substituenten der Gruppe Niederalkyl, Niederalkoxy, Niederalkenyloxy, Niederalkinyloxy, Niederalkylmercapto, Niederalkylsulfinyl, Niederalkylsulfonyl, Halogen und Trifluormethyl, oder durch ein Niederalkylendioxy substituiert ist, wobei die mit "nieder" bezeichneten Gruppen höchstens 7 Kohlenstoffatome enthalten, und ihre Säureadditionssalze herstellt.

3. Verfahren nach Anspruch 1, dadurch gekennzeichnet, dass man Verbindungen der Formel IA

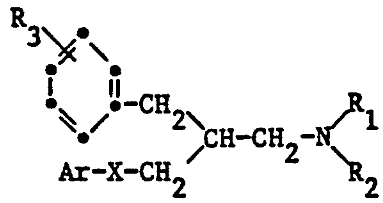 (IA),

worin $R_1$ und $R_2$ unabhängig voneinander Wasserstoff oder Niederalkyl bedeuten, oder $R_1$ und $R_2$ zusammen mit dem Stickstoffatom für Piperidino oder Pyrrolidino, oder für einen fünf- oder sechsgliedrigen gesättigten heterocyclischen Ring stehen, der NH, N-Niederalkyl, O oder S enthält; $R_3$ Wasserstoff, Niederalkyl, Niederalkoxy, Halogen oder Trifluormethyl bedeutet; Ar für einen Phenylrest steht, der durch einen oder zwei Substituenten der Gruppe Niederalkyl, Niederalkoxy, Niederalkenyloxy, Niederalkinyloxy, Halogen oder Trifluormethyl oder durch ein Niederalkylendioxy substituiert ist; und X für O oder S steht; wobei die mit "nieder" bezeichneten Gruppen höchstens 7 Kohlenstoffatome enthalten, und ihre Säureadditionssalze herstellt.

4. Verfahren nach Anspruch 1, dadurch gekennzeichnet, dass man Verbindungen der im Anspruch 3 gezeigten Formel IA, worin $R_1$ und $R_2$ unabhängig voneinander Wasserstoff oder Niederalkyl bedeuten, oder $R_1$ und $R_2$ zusammen mit dem Stickstoffatom für Pyrrolidino oder Piperidino stehen; $R_3$, Ar und X die im Anspruch 3 angegebenen Bedeutungen haben; wobei die mit "nieder" bezeichneten Gruppen höchstens 7 Kohlenstoffatome enthalten, und ihre Säureadditionssalze herstellt.

5. Verfahren nach Anspruch 1, dadurch gekennzeichnet, dass man Verbindungen der Formel IB

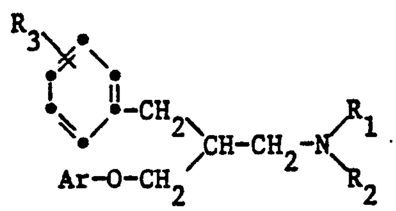 (IB),

worin $R_1$ und $R_2$ unabhängig voneinander Wasserstoff oder Niederalkyl bedeuten; $R_3$ für Wasserstoff, Halogen oder Trifluormethyl steht; Ar Phenyl bedeutet, welches durch einen oder zwei, gleiche oder verschiedene Substituenten der Gruppe Niederalkyl, Niederalkoxy, Niederalkenyloxy, Niederalkylmercapto, Niederalkylsulfinyl, Niederalkylsulfonyl, Halogen oder Trifluormethyl substituiert ist, wobei die mit "nieder" bezeichneten Gruppen höchstens 7 Kohlensoffatome enthalten, und ihre Säureadditionssalze herstellt.

6. Verfahren nach Anspruch 1, dadurch gekennzeichnet, dass man Verbindungen der im Anspruch 1 angegebenen Formel I, worin $R_1$ Wasserstoff oder Methyl ist, $R_2$ für Methyl steht; $R_3$ Wasserstoff, Niederalkyl, Niederalkoxy, Halogen oder Trifluormethyl bedeutet; n für die ganze Zahl 1 oder 2 steht, X Sauerstoff bedeutet und Ar für unsubstituiertes Phenyl steht, wobei die mit "nieder" bezeichneten Gruppen höchstens 7 Kohlenstoffatome erhalten, und ihre Säureadditonsalze herstellt.

7. Verfahren nach Anspruch 1, dadurch gekennzeichnet, dass man N,N-Dimethyl-2-benzyl-3-(4-trifluormethylphenoxy)propylamin und seine Säureadditionssalze herstellt.

8. Verfahren nach Anspruch 1, dadurch gekennzeichnet, dass man N,N-Dimethyl-2-(3-chlorbenzyl)-3-(4-methylmercaptophenoxy)propylamin und seine Säureadditionssalze herstellt.

9. Verfahren nach Anspruch 1, dadurch gekennzeichnet, dass man N-Methyl-2-benzyl-3-(4-trifluormethylphenoxy)propylamin und seine Säureadditionssalze herstellt.

10. Verfahren nach Anspruch 1 zur Herstellung von Verbindungen der im Anspruch 3 gezeigten Formel IA, worin $R_1$ und $R_2$ unabhängig voneinander Wasserstoff oder Niederalkyl bedeuten, oder $R_1$ und $R_2$ zusammen mit dem Stickstoffatom für Piperidino oder Pyrrolidino, oder für einen fünf- oder sechsgliedrigen gesättigten heterocyclischen Ring stehen, der NH, N-Niederalkyl, O oder S enthält; $R_3$ Wasserstoff, Niederalkyl, Niederalkoxy, Halogen oder Trifluormethyl bedeutet; Ar für einen Phenylrest steht, der durch einen oder zwei Substituenten der Gruppe Niederalkyl, Niederalkoxy, Niederalkenyloxy, Niederalkinyloxy, Niederalkylmercapto, Niederalkylsulfinyl, Niederalkylsulfonyl, Halogen oder Trifluormethyl oder durch ein

Niederalkylendioxy substituiert ist; und X für O oder S steht, wobei die mit "nieder" bezeichneten Gruppen höchstens 7 Kohlenstoffatome enthalten, ihren N-Oxiden, ihren Säureadditionssalzen, und ihren N-Niederalkyl oder N-Benzyl quaternären Salzen, dadurch gekennzeichnet, dass man

a) eine Verbindung der Formel II

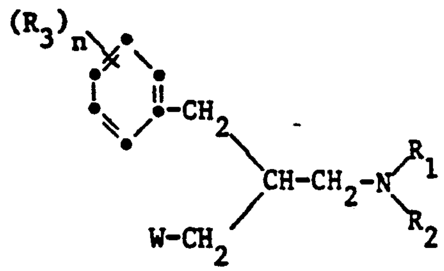

(II)

oder ein Säureadditionssalz davon, mit einer Verbindung der Formel III

Ar - Y (III),

worin $R_1$, $R_2$, $R_3$, n und Ar die oben angegebenen Bedeutungen haben, eines der Symbole W und Y die Gruppe -XH oder XH in der Form eines Metallsalzes bedeutet, worin X die obige Bedeutung hat, und das andere für die Gruppe XH in reaktionsfähig veresterter Form oder als ein N,N'-disubstituiertes Isoharnstoff- oder Isothioharnstoff-Derivat steht, kondensiert, oder

b) ein Amin der Formel IV

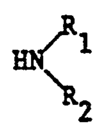

(IV)

oder ein Säureadditionssalz davon, mit einer Verbindung der Formel V

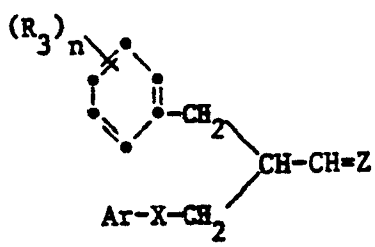

(V),

worin Z ein Wasserstoffatom zusammen mit einer reaktionsfähigen veresterten Hydroxygruppe oder die Oxogrupe bedeutet, und Ar, X, $R_3$ und n die vorher angegebenen Bedeutungen haben alkyliert, und wenn die Gruppe Z Oxo bedeutet wird die Alkylierung unter reduktiven Bedingungen durchgeführt, oder

c) eine Verbindung der Struktur, die zu der oben definierten Verbindung der Formel I analog ist, und mindestens an einem mit dem Amino-Stickstoffatom benachbarten Kohlenstoffatom eine Oxogruppe aufweist, reduziert, oder

d) mehrfach Bindung(en) in einer Verbindung der Struktur, die zu der oben definierten Verbindung der Formel I analog ist, und worin im aliphatischen Teil des Moleküls, inklusive die Aminogruppe, eine oder zwei Doppelbindung(en) vorhanden sind, mit Wasserstoff sättigt oder

e) in einer Verbindung der Formel VI

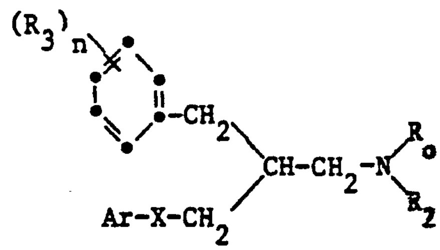

(VI),

worin $R_2$, $R_3$, X n und Ar die oben angegebenen Bedeutungen haben, und $R_0$ eine Aminoschutzgruppe bedeutet, die Gruppe $R_0$ abspaltet, und eine Verbindung der Formel I, worin $R_1$ Wasserstoff ist, erhält, oder

f) eine Verbindung der Formel VII

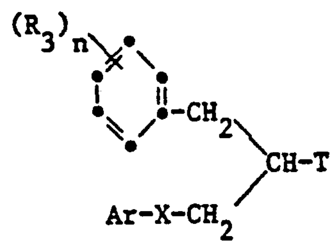

(VII),

worin T Cyan bedeutet, $R_3$, n, X und Ar die obige Bedeutung haben, reduziert, und eine Verbindung der Formel I, worin jedes der Symbole $R_1$ und $R_2$ Wasserstoff bedeutet, erhält, wenn nötig, in allen diesen Verfahren eine störende Gruppe vorübergehend schützt, und die erhaltene Verbindung der Formel I isoliert, und/oder, wenn erwünscht, eine erhaltene Verbindung der Formel I in eine andere Verbindung der Formel I umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

11. Verfahren nach Anspruch 10, dadurch gekennzeichnet, dass man Verbindungen der im Anspruch 10 gezeigten Formel IA, worin $R_1$ und $R_2$ unabhängig voneinander Wasserstoff oder Niederalkyl mit 1 bis 7 Kohlenstoffatomen bedeuten, oder $R_1$ und $R_2$ zusammen mit dem Stickstoffatom für Pyrrolidino oder Piperidino stehen; $R_3$, Ar und X die im Anspruch 10 angegebenen Bedeutungen haben; und ihre Säureadditionssalze herstellt.

12. Verfahren nach Anspruch 10, dadurch gekennzeichnet, dass man Verbindungen der Formel IB

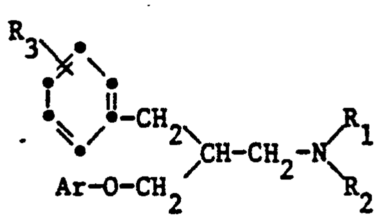

(IB),

worin $R_1$ und $R_2$ unabhängig voneinander Wasserstoff oder Niederalkyl bedeuten; $R_3$ für Wasserstoff, Halogen oder Trifluormethyl steht; Ar Phenyl bedeutet, welches durch einen oder zwei, gleiche oder verschiedene Substituenten der Gruppe Niederalkyl, Niederalkoxy, Niederalkenyloxy, Niederalkylmercapto, Niederalkysulfinyl, Niederalkylsulfonyl, Halogen oder Trifluormethyl substituiert ist, wobei die mit "nieder"

bezeichneten Gruppen höchstens 7 Kohlenstoffatome enthalten, und ihre Säureadditionssalze herstellt.

13. Verfahren nach Anspruch 10, dadurch gekennzeichnet, dass man N,N-Dimethyl-2-benzyl-3-(4-trifluormethylphenoxy)propylamin und seine Säureadditionssalze herstellt.

14. Verfahren nach Anspruch 10, dadurch gekennzeichnet, dass man N,N-Dimethyl-2-(3-chlorbenzyl)-3-(4-methylmercaptophenoxy)propylamin und seine Säureadditionssalze herstellt.

15. Verfahren nach Anspruch 10, dadurch gekennzeichnet, dass man N-Methyl-2-benzyl-3-(4-trifluormethylphenoxy)propylamin und seine Säureadditionssalze herstellt.

16. Verfahren zur Herstellung eines pharmazeutischen Präparates, gekennzeichnet durch die Verarbeitung eines erfindungsgemässen Wirkstoffes nach einem der Ansprüche 1 bis 9, mit einem pharmazeutischen Trägermaterial.

17. Verfahren zur Herstellung eines pharmazeutischen Präparates, gekennzeichnet durch die Verarbeitung eines erfindungsgemässen Wirkstoffes nach einem der Ansprüche 10 bis 15, mit einem pharmazeutischen Trägermaterial.

**Claims**

for the Contracting States: DE, GB, FR, CH, LI, IT, NL, BE, SE, LU.

1. A compound of the general formula I

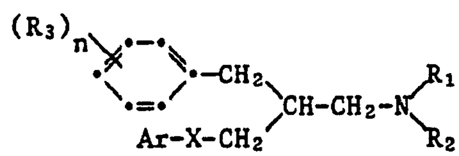

(I)

wherein each of $R_1$ and $R_2$ independently of the other represents hydrogen or lower alkyl; or $R_1$ and $R_2$ together with the adjacent nitrogen atom represent piperidino or pyrrolidino or a five- or six-membered saturated heterocyclic ring containing NH, N-lower alkyl, O or S; $R_3$ represents hydrogen, lower alkyl, lower alkoxy, halogen or trifluoromethyl; Ar represents phenyl or phenyl substituted by one to three substituents selected from the group consisting of lower alkyl, lower alkoxy, lower alkenyloxy, lower alkynyloxy, lower alkylmercapto, lower alkylsulfinyl, lower alkylsulfonyl, halogen and trifluoromethyl, or by one lower alkylenedioxy; and X represents O, S or N-lower alkyl; and n represents an integer 1, 2 or 3; or an N-oxide thereof; an acid addition salt thereof; or an N-lower alkyl or N-benzyl quaternary salt thereof; with the exception of the compound of formula I wherein $R_1$ and $R_2$ represent ethyl, $R_3$ represents hydrogen, Ar represents phenyl, X represents O, and n is 3, or a salt thereof; with the groups qualified by the term "lower" containing at most 7 carbon atoms.

2. A compound of the formula I according to claim 1, wherein each of $R_1$ and $R_2$ independently of the other represents hydrogen or lower alkyl; or $R_1$ and $R_2$ together with the adjacent nitrogen atom represent pyrrolidino or piperidino or a five- or six-membered saturated heterocyclic ring containing NH, N-lower alkyl, O or S; $R_3$ represents hydrogen, lower alkyl, lower alkoxy, halogen or trifluoromethyl; n represents the integer 1 or 2; X represents O, S or N-lower alkyl; Ar represents phenyl substituted by one or two substituents selected from the group consisting of lower alkyl, lower alkoxy, lower alkenyloxy, lower alkynyloxy, lower alkylmercapto, lower alkylsulfinyl, lower alkylsulfonyl, halogen and trifluoromethyl, or by one lower alkylenedioxy; with the groups qualified by the term "lower" containing at most 7 carbon atoms; or an acid addition salt thereof.

3. A compound of the formula IA

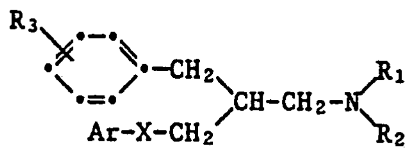

(IA)

wxherein each of $R_1$ and $R_2$ independently of the other represents hydrogen or lower alkyl; or $R_1$ and $R_2$ together with the adjacent nitrogen atom represent piperidino or pyrrolidino or a five- or six-membered saturated heterocyclic ring containing NH, N-lower alkyl, O or S; $R_3$ represents hydrogen, lower alkyl, lower alkoxy, halogen or trifluoromethyl; Ar represents phenyl substituted by one or two substituents selected from the group consisting of lower alkyl, lower alkoxy, lower alkenyloxy, lower alkynyloxy, halogen and trifluoromethyl, or by one lower alkylenedioxy; and X represents O or S; with the groups qualified by the term "lower" containing at most 7 carbon atoms; or an acid addition salt thereof.

4. A compound of the formula IA, wherein each of $R_1$ and $R_2$ independently represents hydrogen or lower alkyl containing 1 to 7 carbon atoms; or $R_1$ and $R_2$ together with the adjacent nitrogen atom represent pyrrolidino or piperidino; and $R_3$, Ar and X are as defined in claim 3; or an acid addition salt thereof.

5. A compound of the formula IB

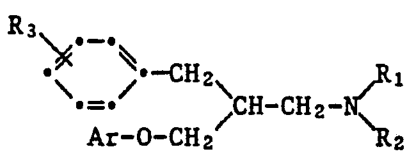

(IB)

wherein each of $R_1$ and $R_2$ independently represents hydrogen or lower alkyl; $R_3$ represents hydrogen, halogen or trifluoromethyl; Ar represents phenyl monosubstituted or disubstituted by identical or different substituents selected from lower alkyl, lower alkoxy, lower alkenyloxy, lower alkylmercapto, lower alkylsulfinyl, lower alkylsulfonyl, halogen and trifluoromethyl; with the groups qualified by the term "lower" containing at most 7 carbon atoms; or an acid addition salt thereof.

6. A compound of the formula 1 as shown in claim 1, wherein $R_1$ represents hydrogen or methyl; $R_2$ is methyl; $R_3$ represents hydrogen, lower alkyl, lower alkoxy, halogen or trifluoromethyl; n is the integer 1 or 2; X represents O and Ar is unsubstituted phenyl; with the groups qualified by the term "lower" containing at most 7 carbon atoms; or an acid addition salt thereof.

7. N,N-Dimethyl-2-benzyl-3-(4-trifluoromethylphenoxy)propylamine or an acid addition salt thereof.

8. N,N-Dimethyl-2-(3-chlorobenzyl)-3-(4-methylmercaptophenoxy)propylamine or an acid addition salt thereof.

9. N-Methyl-2-benzyl-3-(4-trifluoromethylphenoxy)propylamine or an acid addition salt thereof.

10. A pharmaceutical composition containing a compound as claimed in any one of claims 1, 2 and 6, in admixture or conjunction with a pharmaceutically suitable carrier.

11. A pharmaceutical composition containing a compound as claimed in any one of claims 3, 4, 5 and 7 to 9 in admixture or conjunction with a pharmaceutically suitable carrier.

12. A compound as claimed in any one of claims 1, 2 and 6 for use in a therapeutic method of treating the human or animal body.

13. A compound as claimed in any one of claims 3, 4, 5 and 7 to 9 for use in a therapeutic method of treating the human or animal body.

14. A compound as claimed in any one of claims 1, 2 and 6 for use as antidepressant.

15. A compound as claimed in any one of claims 3, 4, 5 and 7 to 9 for use as antidepressant.

16. Use of a compound as claimed in any one of claims 1, 2 and 6 for the preparation of pharmaceutical compositions.

17. Use of a compound as claimed in any one of claims 3, 4, 5 and 7 to 9 for the preparation of

pharmaceutical compositions.

18. A process for the preparation of a compound of formula 1 as claimed in claim 1 or of an N-oxide thereof, an acid addition salt thereof, or an N-lower alkyl or N-benzyl quaternary salt thereof, which process comprises

a) condensing a compound of the formula II

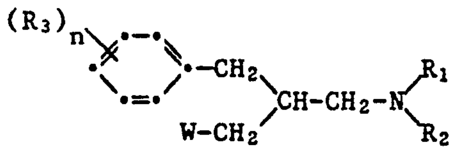

(II)

or an acid addition salt thereof, with a compound of the formula III

Ar-Y (III)

wherein $R_1$, $R_2$, $R_3$, n and Ar are as defined above, one of the symbols W and Y represents a group XH or XH in a metal salt form, in which X is as defined above, and the other one represents the group XH in a reactive esterified form thereof or as an N,N'-disubstituted isourea or isothiourea derivative; or

b) alkylating an amine of the formula IV

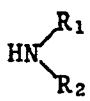

(IV)

or an acid addition salt thereof, with a compound of the formula V

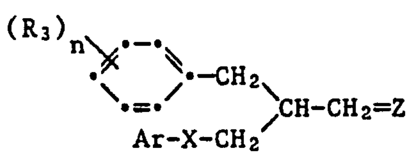

(V)

in which Z is a hydrogen atom together with a reactive esterified hydroxyl group or the oxo group and Ar, X, $R_3$ and n are as defined above, and if Z is the oxo group, said alkylation is carried out under reductive conditions; or

c) reducing a compound structurally analogous to a compound of formula I as defined above, wherein an oxo group is present on at least one carbon atom adjacent to the amino nitrogen; or

d) saturating with hydrogen a multiple bond or multiple bonds in a compound structurally analogous to the compound of formula I as defined above and wherein one or two double bonds are present in the aliphatic moiety of the molecule inclusive of the amino group; or

e) removing the radical $R_0$ from a compound of the formula VI

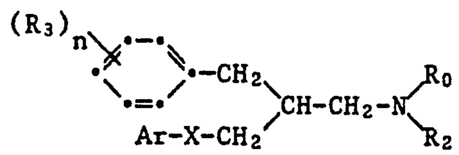

(VI)

wherein $R_2$, $R_3$, X, n and Ar are as defined above and $R_0$ is an amino protective group, to obtain a compound of formula I in which $R_1$ is hydrogen; or

f) reducing a compound of the formula VII

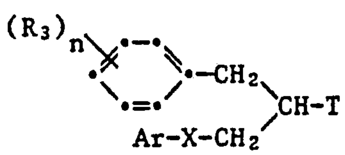

(VII)

wherein T represents cyano or azidomethyl; and $R_3$, X, n and Ar are as defined above to obtain a compound of formula I wherein $R_1$ and $R_2$ are hydrogen; or

g) condensing a compound of the formula VIIA

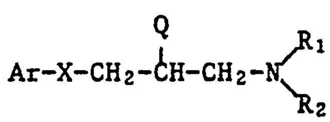

(VIIA)

with a compound of the formula VIIB

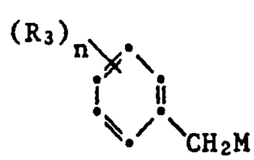

(VIIB)

wherein one of the groups Q and M represents a removable group, and the other represents a metal or halometal radical, and Ar, X, $R_1$ to $R_3$ and n are as defined above, if necessary while temporarily protecting any interfering reactive group in all these processes, and isolating the resultant compound of formula I; and/or, if required, converting a resultant compound of formula I into another compound of formula I, and/or, if required, converting a resultant free compound into a salt or a resultant salt into the free compound or into another salt, and/or, if required, resolving a mixture of isomers or racemates obtained into the single isomers or racemates, and/or, if required, resolving a racemate obtained into the optical antipodes.

## Claims

for the Contracting State: AT

1. A process for the preparation of a novel propylamine derivative of the general formula I

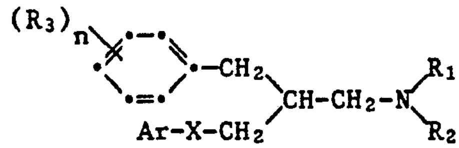

(I)

wherein each of $R_1$ and $R_2$ independently of the other represents hydrogen or lower alkyl; or $R_1$ and $R_2$ together with the adjacent nitrogen atom represent piperidino or pyrrolidino or a five- or six-membered saturated heterocyclic ring containing NH, N-lower alkyl, O or S; $R_3$ represents hydrogen, lower alkyl, lower alkoxy, halogen or trifluoromethyl; Ar represents phenyl or phenyl substituted by one to three substituents selected from the group consisting of lower alkyl, lower alkoxy, lower alkenyloxy, lower alkynyloxy, lower alkylmercapto, lower alkylsulfinyl, lower alkylsulfonyl, halogen and trifluoromethyl, or by one lower alkylenedioxy; and X represents O, S or N-lower alkyl; and n represents an integer 1, 2 or 3; or of an N-oxide thereof; an acid addition salt thereof; or an N-lower alkyl or N-benzyl quaternary salt thereof; with the exception of the compound of formula I wherein $R_1$ and $R_2$ represent ethyl, $R_3$ represents hydrogen, Ar represents phenyl, X represents O, and n is 3, or a salt thereof; with the groups qualified by the term "lower" containing at most 7 carbon atoms; which process comprises

a) condensing a compound of the formula II

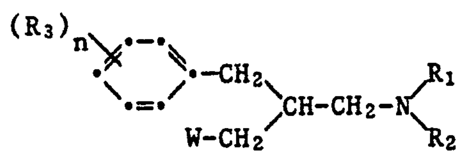

(II)

or an acid addition salt thereof, with a compound of the formula III

Ar-Y (III)

wherein $R_1$, $R_2$, $R_3$, n and Ar are as defined above, one of the symbols W and Y represents a group XH or XH in a metal salt form, in which X is as defined above, and the other one represents the group XH in a reactive esterified form thereof or as an N,N'-disubstituted isourea or isothiourea derivative; or

b) alkylating an amine of the formula IV

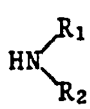

(IV)

or an acid addition salt thereof, with a compound of the formula V

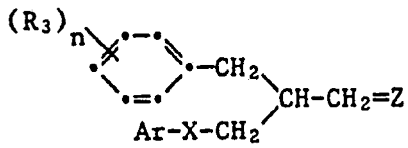

(V)

in which Z is a hydrogen atom together with a reactive esterified hydroxyl group or the oxo group and Ar, X, $R_3$ and n are as defined above, and if Z is the oxo group, said alkylation is carried out under reductive conditions; or

c) reducing a compound structurally analogous to a compound of formula I as defined above, wherein an oxo group is present on at least one carbon atom adjacent to the amino nitrogen; or

d) saturating with hydrogen a multiple bond or multiple bonds in a compound structurally analogous to the compound of formula I as defined above and wherein one or two double bonds are present in the aliphatic moiety of the molecule inclusive of the amino group; or

e) removing the radical $R_0$ from a compound of the formula VI

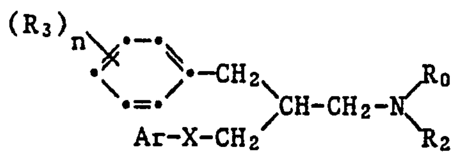

(VI)

wherein $R_2$, $R_3$, X, n and Ar are as defined above and $R_0$ is an amino protective group, to obtain a compound of formula I in which $R_1$ is hydrogen; or

f) reducing a compound of the formula VII

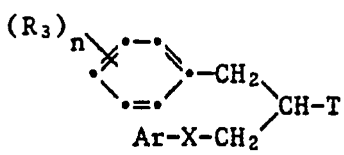

(VII)

wherein T represents cyano or azidomethyl; and $R_3$, X, n and Ar are as defined above to obtain a compound of formula I wherein $R_1$ and $R_2$ are hydrogen; or

g) condensing a compound of the formula VIIA

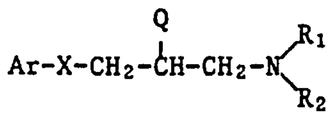 (IIA)

with a compound of the formula VIIB

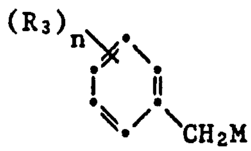 (VIIB)

wherein one of the groups Q and M represents a removable group, and the other represents a metal or halometal radical, and Ar, X, $R_1$ to $R_3$ and n are as defined above, if necessary while temporarily protecting any interfering reactive group in all these processes, and isolating the resultant compound of formula I; and/or, if required, converting a resultant compound of formula I into another compound of formula I, and/or, if required, converting a resultant free compound into a salt or a resultant salt into the free compound or into another salt, and/or, if required, resolving a mixture of isomers or racemates obtained into the single isomers or racemates, and/or, if required, resolving a racemate obtained into the optical antipodes.

2. A process according to claim 1, which comprises preparing a compound of formula I, wherein each of $R_1$ and $R_2$ independently of the other represents hydrogen or lower alkyl; or $R_1$ and $R_2$ together with the adjacent nitrogen atom represent pyrrolidino or piperidino or a five- or six-membered saturated heterocyclic ring containing NH, N-lower alkyl, O or S; $R_3$ represents hydrogen, lower alkyl, lower alkoxy, halogen or trifluoromethyl; n represents the integer 1 or 2; X represents O, S or N-lower alkyl; Ar represents phenyl substituted by one or two substituents selected from the group consisting of lower alkyl, lower alkoxy, lower alkenyloxy, lower alkynyloxy, lower alkylmercapto, lower alkylsulfinyl, lower alkylsulfonyl, halogen and trifluoromethyl, or by one lower alkylenedioxy; with the groups qualified by the term "lower" containing at most 7 carbon atoms; or an acid addition salt thereof.

3. A process according to claim 1, which comprises preparing a compound of the formula IA

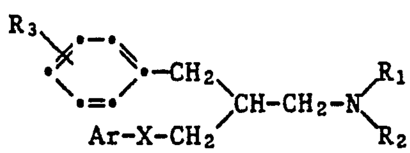 (IA)

wherein each of $R_1$ and $R_2$ independently of the other represents hydrogen or lower alkyl; or $R_1$ and $R_2$ together with the adjacent nitrogen atom represent piperidino or pyrrolidino or a five- or six-membered saturated heterocyclic ring containing NH, N-lower alkyl, O or S; $R_3$ represents hydrogen, lower alkyl, lower alkoxy, halogen or triflourmethyl; Ar represents phenyl substituted by one or two substituents selected from the group consisting of lower alkyl, lower alkoxy, lower alkenyloxy, lower alkynyloxy, halogen and trifluoromethyl, or by one lower alkylenedioxy; and X represents O or S; with the groups qualified by the term "lower" containing at most 7 carbon atoms; or an acid addition salt thereof.

4. A process according to claim 1, which comprises preparing a compound of the formula IA as shown in claim 3, wherein each of $R_1$ and $R_2$ independently represents hydrogen or lower alkyl or; $R_1$ and $R_2$ together with the adjacent nitrogen atom represent pyrrolidino or piperidino; and $R_3$, Ar and X are as defined in claim 3; with the groups qualified by the term "lower" containing at most 7 carbon atoms; or an acid addition salt thereof.

5. A process according to claim 1, which comprises preparing a compound of the formula IB

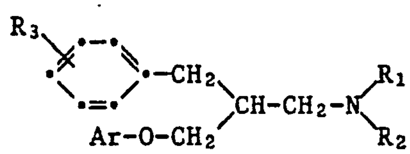

(IB)

wherein each of $R_1$ and $R_2$ independently represents hydrogen or lower alkyl; $R_3$ represents hydrogen, halogen or trifluoromethyl; Ar represents phenyl monosubstituted or disubstituted by identical or different substituents selected from lower alkyl, lower alkoxy, lower alkenyloxy, lower alkylmercapto, lower alkylsulfinyl, lower alkylsulfonyl, halogen and trifluoromethyl; with the groups qualified by the term "lower" containing at most 7 carbon atoms; or an acid addition salt thereof.

6. A process according to claim 1, which comprises preparing a compound of the formula I as shown in claim 1, wherein $R_1$ represents hydrogen or methyl; $R_2$ is methyl; $R_3$ represents hydrogen, lower alkyl, lower alkoxy, halogen or trifluoromethyl; n is the integer 1 or 2; X represents O and Ar is unsubstituted phenyl; with the groups qualified by the term "lower" containing at most 7 carbon atoms; or an acid addition salt thereof.

7. A process according to claim 1, which comprises preparing N,N-dimethyl-2-benzyl-3-(4-trifluoromethylphenoxy)propylamine or an acid addition salt thereof.

8. A process according to claim 1, which comprises preparing N,N-dimethyl-2-(3-chlorobenzyl)-3-(4-methylmercaptophenoxy)propylamine or an acid addition salt thereof.

9. A process according to claim 1, which comprises preparing N-methyl-2-benzyl-3-(4-trifluoromethylphenoxy)propylamine or an acid addition salt thereof.

10. A process according to claim 1 for the preparation of a compound of the formula IA as shown in claim 3, wherein each of $R_1$ and $R_2$ independently of the other represents hydrogen or lower alkyl; or $R_1$ and $R_2$ together with the adjacent nitrogen atom represent piperidino or pyrrolidino or a five- or six-membered saturated heterocyclic ring containing NH, N-lower alkyl, O or S; $R_3$ represents hydrogen, lower alkyl, lower alkoxy, halogen or trifluoromethyl; Ar represents phenyl substituted by one or two substituents selected from the group consisting of lower alkyl, lower alkoxy, lower alkenyloxy, lower alkynyloxy, lower alkylmercapto, lower alkylsulfinyl, lower alkylsulfonyl, halogen and trifluoromethyl, or by one lower alkylenedioxy; and X represents O or S; or of an N-oxide thereof; an acid addition salt thereof; or an N-lower alkyl or N-benzyl quaternary salt thereof; with the groups qualified by the term "lower" containing at most 7 carbon atoms; which process comprises

a) condensing a compound of the formula II

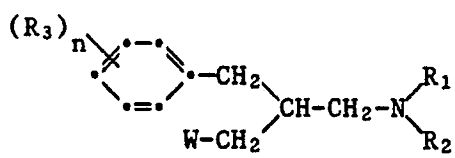

(II)

or an acid addition salt thereof, with a compound of the formula III

Ar-Y (III)

wherein $R_1$, $R_2$, $R_3$, n and Ar are as defined above, one of the symbols W and Y represents a group XH or XH in a metal salt form, in which X is as defined above; and the other one represents the group XH in a reactive esterified form thereof or as an N,N'-disubstituted isourea or isothiourea derivative; or

b) alkylating an amine of the formula IV

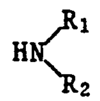

(IV)

or an acid addition salt thereof, with a compound of the formula V

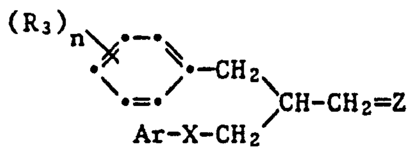

(V)

in which Z is a hydrogen atom together with a reactive esterified hydroxyl group or the oxo group and Ar, X, $R_3$ and n are as defined above, and if Z is the oxo group, said alkylation is carried out under reductive conditions; or

c) reducing a compound structurally analogous to a compound of formula I as defined above, wherein an oxo group is present on at least one carbon atom adjacent to the amino nitrogen; or

d) saturating with hydrogen a multiple bond or multiple bonds in a compound structurally analogous to the compound of formula I as defined above and wherein one or two double bonds are present in the aliphatic moiety of the molecule inclusive of the amino group; or

e) removing the radical $R_0$ from a compound of the formula VI

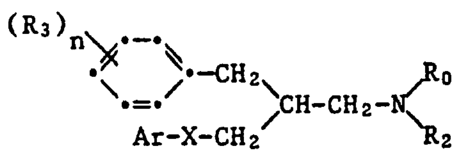

(VI)

wherein $R_2$, $R_3$, X, n and Ar are as defined above and $R_0$ is an amino protective group, to obtain a compound of formula O in which $R_1$ is hydrogen; or

f) reducing a compound of the formula VII

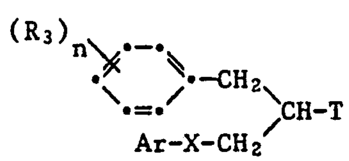

(VII)

wherein T represents cyano; and $R_3$, X, n and Ar are as defined above to obtain a compound of formula I wherein $R_1$ and $R_2$ are hydrogen; if necessary while temporarily protecting any interfering reactive group in all

these processes, and isolating the resultant compound of formula I; and/or, if required, converting a resultant compound of formula I into another compound of formula I, and/or, if required, converting a resultant free compound into a salt or a resultant salt into the free compound or into another salt, and/or, if required, resolving a mixture of isomers or racemates obtained into the single isomers or racemates, and/or, if required, resolving a racemate obtained into the optical antipodes.

11. A process according to claim 10, which comprises preparing a compound of the formula IA as shown in claim 10, wherein each of $R_1$ and $R_2$ independently represents hydrogen or lower alkyl containing 1 to 7 carbon atoms; or $R_1$ and $R_2$ together with the adjacent nitrogen atom represent pyrrolidino or piperidino; and $R_3$, Ar and X are as defined in claim 10; or an acid addition salt thereof.

12. A process according to claim 10, which comprises preparing a compound of the formula IB

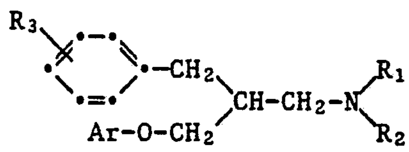

(IB)

wherein each of $R_1$ and $R_2$ independently represents hydrogen or lower alkyl; $R_3$ represents hydrogen, halogen or trifluoromethyl; Ar represents phenyl monosubstituted or disubstituted by identical or different substituents selected from lower alkyl, lower alkoxy, lower alkenyloxy, lower alkylmercapto, lower alkylsulfinyl, lower alkylsulfonyl, halogen and trifluoromethyl; with the groups qualified by the term "lower" containing at most 7 carbon atoms; or an acid addition salt thereof.

13. A process according to claim 10, which comprises preparing N,N-dimethyl-2-benzyl-3-(4-trifluoromethylphenoxy)propylamine or an acid addition salt thereof.

14. A process according to claim 10, which comprises preparing N,N-dimethyl-2-(3-chlorobenzyl)-3-(4-methylmercaptophenoxy)propylamine or an acid addition salt thereof.

15. A process according to claim 10, which comprises preparing N-methyl-2-benzyl-3-(4-trifluoromethylphenoxy)propylamine or an acid addition salt thereof.

16. A process for the preparation of a pharmaceutical composition, which process comprises combining a compound obtained by a process according to any one of claims 1 to 9 with a pharmaceutically suitable carrier.

17. A process for the preparation of a pharmaceutical composition, which process comprises combining a compound obtained by a process according to any one of claims 10 to 15 with a pharmaceutically suitable carrier.

**Revendications**

pour les Etats contractants: BE, CH, DE, FR, GB, IT, LI, LU, NL, SE.

1. Composés de formule générale I

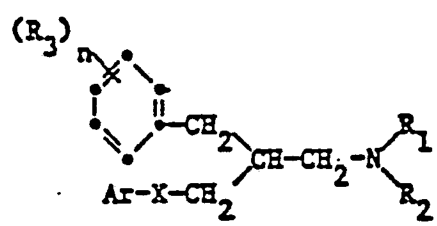

(I),

où chacun des symboles $R_1$ et $R_2$ représente un hydrogène ou un alcoyle inférieur, ou bien $R_1$ et $R_2$ représentent ensemble avec l'atome d'azote un pipéridino ou un pyrrolidino, ou un noyau hétérocyclique saturé à 5 ou 6 chaînons, qui contient NH, N-alcoyle inférieur, O ou S; $R_3$ représente un hydrogène, alcoyle inférieur, alcoxy inférieur, halogène ou trifluorométhyle; Ar représente un phényle éventuellement substitué par de 1 à 3 substituants du groupe alcoyle inférieur, alcoxy inférieur, alcényloxy inférieur, alcynyloxy inférieur, alcoyle inférieur-mercapto, alcoyle inférieur-sulfinyle, alcoyle inférieur-sulfonyle, halogène et trifluorométhyle, ou par un alcoylène inférieur-dioxy; X représente un atome d'oxygène, un atome de soufre ou un N-alcoyle inférieur, et n représente le nombre 1, 2 ou 3; leur N-oxyde; leurs sels d'addition d'acide, et leurs sels quaternaire de N-alcoyle inférieur ou de N-benzyle, à l'exception du composé de formule I où chacun des symboles $R_1$ et $R_2$ représente un éthyle, $R_3$ représente un hydrogène, Ar représente un phényle, X représente un atome d'oxygène, et n vaut 3, où les groupes désignés par "inférieur" contiennent au plus 7 atomes de carbone, et leurs sels.

2. Composés selon la revendication 1, où $R_1$ et $R_2$ représentent indépendamment l'un de l'autre un hydrogène ou un alcoyle inférieur, ou $R_1$ et $R_2$ représentent ensemble avec l'atome d'azote un pyrrolidino ou un pipéridino ou un noyau hétérocyclique saturé à 5 ou 6 chaînons, qui contient NH, N-alcoyle inférieur, O ou S, $R_3$ représente un hydrogène, alcoyle inférieur, alcoxy inférieur, halogène ou trifluorométhyle, n représente le nombre 1 ou 2, X représente un oxygène, un soufre ou un N-alcoyle inférieur, Ar représente un radical phényle, qui est substitué par un ou deux substituants du groupe alcoyle inférieur, alcoxy inférieur, alcényloxy inférieur, alcynyloxy inférieur, alcoyle inférieur-mercapto, alcoyle inférieur-sulfinyle, alcoyle inférieur-sulfonyle, halogène et trifluorométhyle ou par un alcoylène inférieur-dioxy, où les groupes désignés par "inférieur" contiennent au plus 7 atomes de carbone et leurs sels d'addition d'acide.

3. Composés de formule IA

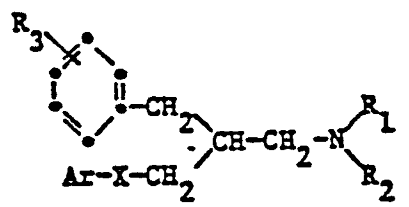

(IA),

où $R_1$ et $R_2$ représentent indépendamment l'un de l'autre un hydrogène ou un alcoyle inférieur ou $R_1$ et $R_2$ représentent avec l'atome d'azote un pipéridino ou un pyrrolidino ou un noyau hétérocyclique saturé à 5 ou 6 chaînons, qui contient NH, N-alcoyle inférieur, O ou S; $R_3$ représente un hydrogène, alcoyle inférieur, alcoxy inférieur, halogène ou trifluorométhyle; Ar représente un radical phényle, qui est substitué par un ou deux substituants du groupe alcoyle inférieur, alcoxy inférieur, alcényloxy inférieur, alcynyloxy inférieur, halogène ou trifluorométhyle ou par un alcoylène inférieur-dioxy; et X représente O ou S, où les groupes désignés par "inférieur" contiennent au plus 7 atomes de carbone, et leurs sels d'addition d'acide.

4. Composés de formule IA, où $R_1$ et $R_2$ représentent indépendamment l'un de l'autre un hydrogène ou un alcoyle inférieur ayant de 1 à 7 atomes de carbone ou $R_1$ et $R_2$ représentent ensemble avec l'atome d'azote un pyrrolidino ou un pipéridino; $R_3$, Ar et X ont les significations données dans la revendication 3; et leurs sels d'addition d'acide.

5. Composés de formule IB

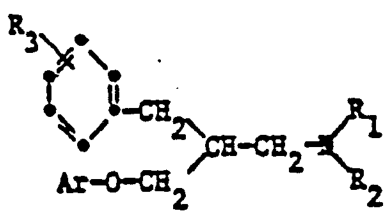

(IB),

où $R_1$ et $R_2$ représentent indépendamment l'un de l'autre un hydrogène ou un alcoyle inférieur; $R_3$ représente un hydrogène, un halogène ou un trifluorométhyle; Ar représente un phényle, qui est substitué par 1 ou 2 substituants identiques ou différents du groupe alcoyle inférieur, alcoxy inférieur, alcényloxy inférieur, alcoyle inférieur-mercapto, alcoyle inférieur-sulfinyle, alcoyle inférieur-sulfonyle, halogène ou trifluorométhyle, où les groupes designés par "inférieur" contiennent au plus 7 atomes de carbone; et leurs sels d'addition d'acide.

6. Composés de formule I présentés dans la revendication 1, où $R_1$ est un hydrogène ou un méthyle, $R_2$ represente un méthyle; $R_3$ représente un hydrogène, alcoyle inférieur, alcoxy inférieur, halogène ou trifluorométhyle; n représente le nombre entier 1 ou 2, X représente un oxygène et Ar représente un phényle non substitué où les groupes désignés par "inférieur" contiennent au plus 7 atomes de carbone, et leurs sels d'addition d'acide.

7. N,N-diméthyl-2-benzyl-3-(4-trifluorométhylphénoxy)-propylamine et ses sels d'addition d'acide.

8. N,N-diméthyl-2-(3-chlorobenzyl)-3-(4-méthylmercaptophénoxy)propylamine et ses sels d'addition d'acide.

9. N-méthyl-2-benzyl-3-(4-trifluorométhylphénoxy)propylamine et ses sels d'addition d'acide.

10. Préparations pharmaceutiques contenant des composés des revendications 1, 2 et 6, avec un support pharmaceutique.

11. Préparations pharmaceutiques contenant des composés des revendications 3, 4, 5 et 7 à 9, avec un support pharmaceutique.

12. Les composés mentionnés dans les revendications 1, 2 et 6 aux fins d'application dans un procédé de traitement thérapeutique du corps humain ou animal.

13. Les composés mentionnés dans les revendications 3, 4, 5 et 7 à 9, aux fins d'application dans un procédé de traitement thérapeutique du corps humain ou animal.

14. Les composés mentionnés dans les revendications 1, 2 et 6 aux fins d'application comme antidépresseurs.

15. Les composés mentionnés dans les revendications 3, 4, 5 et 7 à 9, aux fins d'application comme antidépresseurs.

16. Application des composés mentionnés dans les revendications 1, 2 et 6, à la préparation de préparations pharmaceutiques.

17. Application des composés mentionnés dans les renvendications 3, 4, 5 et 7 à 9, à la préparation de préparations pharmaceutlques.

18. Procédé de préparation des composés de formule I mentionnés dans la revendication 1, de leurs N-oxydes, sels d'addition d'acide et sels quaternaire de N-alcoyle inférieur et N-benzyle, caractérisé en ce que

a) on condense un composé de formule II

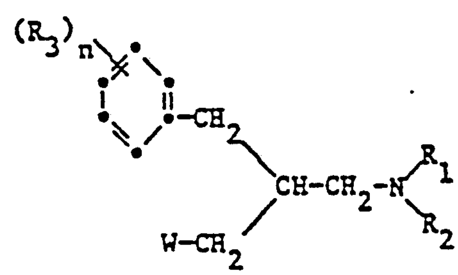

(II)

ou un de ses sels d'addition d'acide, avec un composé de formule III

Ar - Y (III)

ou $R_1$, $R_2$, $R_3$, n et Ar ont les significations données ci-dessus, l'un des symboles W et Y représente le groupe -XH ou XH sous la forme d'un sel métallique où X a la signification donnée ci-dessus, et l'autre représente le groupe XH sous forme estérifiée réactive ou sous la forme d'un dérivé d'iso-urée ou d'isothio-urée N,N'-disubstitué ou

b) on alcoyle une amine de formule IV

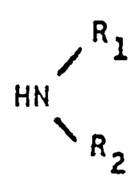

(IV)

ou un de ses sels d'addition d'acide, avec un composé de formule V

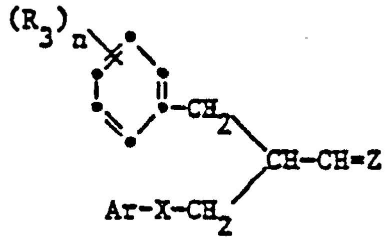 (V),

où Z représente un atome d'hydrogène avec un groupe estérifié réactif ou le groupe oxo, et Ar, X, $R_3$ et n ont les significations données ci-dessus, où lorsque Z représente le groupe oxo, l'alcoylation est effectuée dans des conditions inductrices, ou

c) on réduit un composé de structure qui est analogue à celle du composé de formule I défini ci-dessus, et présente au moins un groupe oxo sur un atome de carbone voisin de l'atome d'azote d'amino, ou

d) on sature avec de l'hydrogène la ou les liaisons multiples dans un composé de structure qui est analogue à celle du composé de formule I défini ci-dessus et ou dans la partie aliphatique de la molécule, y compris le groupe amino, une ou deux doubles liaisons sont présentes, ou

e) Dans un composé de formule VI

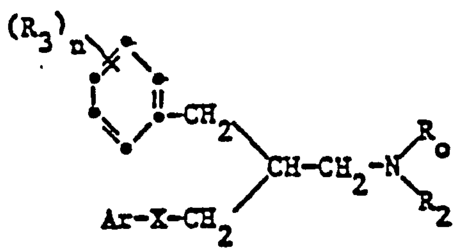 (VI),

où $R_2$, $R_3$, X, n et Ar ont les significations données ci-dessus et $R_0$ représente un groupe protecteur d'amino, on sépare le groupe $R_0$ et on obtient un composé de formule I où $R_1$ est un hydrogène, ou

f) on réduit un composé de formule VII

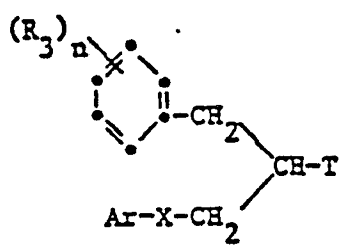 (VII),

où T représente un cyano ou un azidométhyle, $R_3$, n, X et Ar ont la signification donnée ci-dessus et l'on obtient un composé de formule I ou chacun des symboles $R_1$ ou $R_2$ représente un hydrogène, ou

g) on condense un composé de formule VIIA

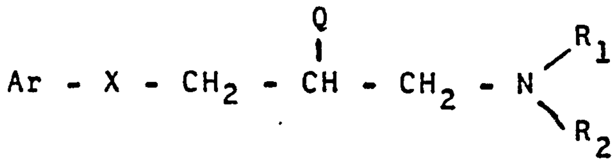 (VIIA)

avec un composé de formule VIIB

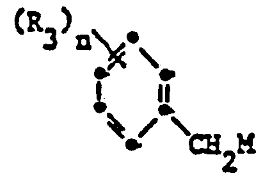

(VIIB),

ou l'un des groupes M et Q représente un groupe séparable, et l'autre est un métal ou un groupe halogène-métal, et Ar, X, $R_1$, $R_3$ et n ont les significations donnés ci-dessus, si nécessaire, on protège temporairement dans tous ce procédés un groupe gênant, et l'on isole le composé de formule I obtenu et/ou, si on le désire, on transforme un composé de formule I, obtenu en un autre composé de formule I, et/ou, si on le désire, on transforme un composé libre obtenu en un sel ou un sel obtenu en le composé libre ou en un autre sel, et/ou, si on le désire, on sépare un mélange d'isomères ou de racémates obtenu en les isomères ou racémates isolés et/ou, si on le désire, on sépare les racémates obtenus en les antipodes optiques.

**Revendications**

pour l'Etat contractant AT.

1. Procédé de préparation de nouveaux dérivés de propylamine de forme générale I

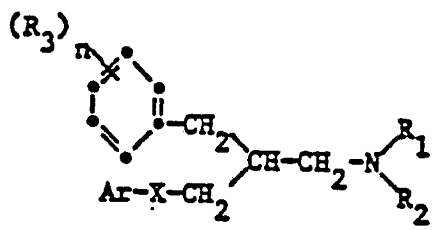

(I),

où chacun des symboles $R_1$ et $R_2$ représente un hydrogène ou un alcoyle inférieur, ou bien $R_1$ et $R_2$ représentent ensemble avec l'atome d'azote un pipéridino ou un pyrrolidino, ou un noyau hétérocyclique saturé à 5 ou 6 chaînons, qui contient NH, N-alcoyle inférieur, O ou S; $R_3$ représente un hydrogène, alcoyle inférieur, alcoxy inférieur, halogène ou trifluorométhyle; Ar représente un phényle éventuellement substitué par de 1 à 3 substituants du groupe alcoyle inférieur, alcoxy inférieur, alcényloxy inférieur, alcynyloxy inférieur, alcoyle inférieur-mercapto, alcoyle inférieur-sulfinyle, alcoyle inférieur-sulfonyle, halogène et trifluorométhyle, ou par un alcoylène inférieur-dioxy; X représente un atome d'oxygène, un atome de soufre ou un N-alcoyle inférieur, et n représente le nombre 1, 2 ou 3; leur N-oxyde; leurs sels d'addition d'acide, et leurs sels quaternaire de N-alcoyle inférieur ou de N-benzyle, à l'exception du composé de formule I où chacun des symboles $R_1$ et $R_2$ représente un éthyle, $R_3$ représente un hydrogène, Ar représente un phényle, X représente un atome d'oxygène, et n vaut 3, où les groupes désignés par "inférieur" contiennent au plus 7 atomes de carbone, et leurs sels, caractérisé en ce que

a) on condense un composé de formule II

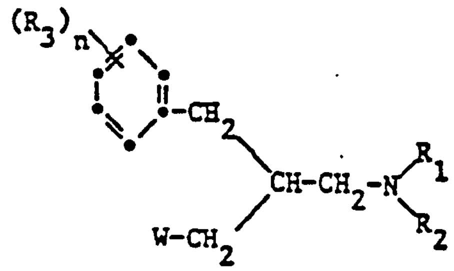

(II)

ou un de ses sels d'addition d'acide, avec un composé de formule III

Ar -Y (III)

où $R_1$, $R_2$, $R_3$, n et Ar ont les significations données ci-dessus, l'un des symboles W et Y représente le groupe -XH ou XH sous la forme d'une sel métallique où X a la signification donnée ci-dessus, et l'autre représente le groupe XH sous forme estérifiée réactive ou sous la forme d'un dérivé d'iso-urée ou d'isothio-urée N,N'-disubstitué ou

b) on alcoyle une amine de formule IV

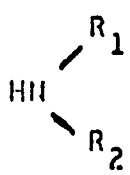

(IV)

ou un de ses sels d'addition d'acide, avec un composé de formule V

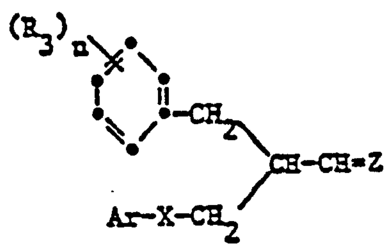

(V),

où Z représente un atome d'hydrogène avec un groupe estérifié réactif ou le groupe oxo, et Ar, X, $R_3$ et n ont les significations données ci-dessus, où lorsque Z représente le groupe oxo, l'alcoylation est effectuée dans des conditions inductrices, ou

c) on réduit un composé de structure qui est analogue à celle du composé de formule I défini ci-dessus, et présente au moins un groupe oxo sur un atome de carbone voisin de l'atome d'azote d'amino, ou

d) on sature avec de l'hydrogène la ou les liaisons multiples dans un composé de structure qui est analogue à celle du composé de formule I défini ci-dessus et ou dans la partie aliphatique de la molécule, y compris le groupe amino, une ou deux doubles liaisons sont présentes, ou

e) dans un composé de formule VI

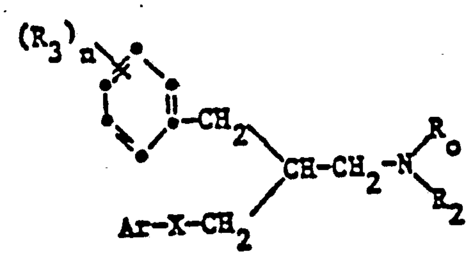

(VI),

où $R_2$, $R_3$, X, n et Ar ont les significations données ci-dessus et $R_0$ représente un groupe protecteur d'amino, on sépare le groupe $R_0$ et on obtient un composé de formule I, où $R_1$ est un hydrogène, ou

f) on réduit un composé de formule VII

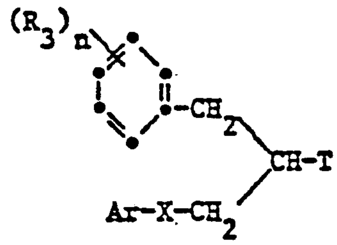

(VII),

où T représente un cyano où un azidométhyle, $R_3$, n, et Ar ont la signification donnée ci-dessus et l'on obtient un composé de formule I où chacun des symboles $R_1$ ou $R_2$ représente un hydrogène, ou

g) on condense un composé de formule VIIA

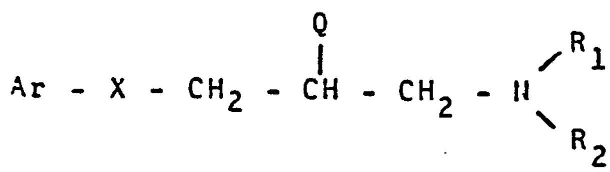

avec un composé de formule VIIB

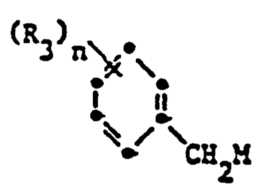

(VIIB),

où l'un des groupes M et Q représente un groupe séparable et l'autre est un métal ou un groupe halogène-métal, et Ar, X, $R_1$, $R_3$ et n ont les significations données ci-dessus, si nécessaire, on protège temporairement dans tous ces procédés un groupe gênant, et l'on isole le composé de formule I obtenu et/ou, si on le désire, on transforme un composé de formule I obtenu en un autre composé de formule I, et/ou, si on le désire, on transforme un composé libre obtenu en un sel ou un sel obtenu en le composé libre ou en un autre sel, et/ou, si on le désire, on sépare un mélange d'isomères ou de racémates obtenu en les isomères ou racémates isolés et/ou, si on le désire, on sépare les racémates obtenus en les antipodes optiques.

2. Procédé selon la revendication 1, caractérisé en ce qu'on prépare des composés de formule I où $R_1$ et $R_2$ représentent indépendamment l'un de l'autre un hydrogène ou alcoyle inférieur, ou $R_1$ et $R_2$ représentent ensemble avec l'atome d'azote un pyrrolidino ou un pipéridino ou un noyau hétérocyclique saturé à 5 ou 6

chaînons, qui contient NH, N-alcoyle inférieur, O ou S, $R_3$ représente un hydrogène, alcoyle inférieur, alcoxy inférieur, halogène ou triflourméthyle, n représente le nombre 1 ou 2, X représente un oxygène, un soufre ou un N-alcoyle inférieur, Ar représente un radical phényle, qui est substitué par un ou deux substituants du groupe alcoyle inférieur, alcoxy inférieur, alcényloxy inférieur, alcynyloxy inférieur, alcoyle inférieur-mercapto, alcoyle inférieur-sulfinyle, alcoyle inférieur-sulfonyle, halogène et trifluorométhyle ou par un alcoylène inférieur-dioxy, où les groupes désignés par "inférieur" contiennent au plus 7 atomes de carbone et leurs sels d'addition d'acide.

3. Procédé selon la revendication 1, caractérisé en ce qu'on prépare des composés de formule IA

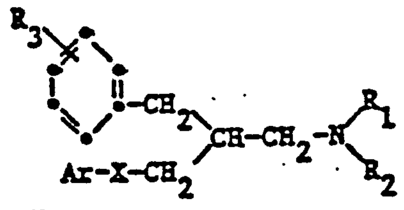

(IA),

où $R_1$ et $R_2$ représentent independamment l'un de l'autre un hydrogène ou un alcoyle inférieur ou $R_1$ et $R_2$ représentent avec l'atome d'azote un pipéridino ou un pyrrolidino ou un noyau hétérocyclique saturé à 5 ou 6 chaînons, qui contient NH, N-alcoyle inférieur, O ou S; $R_3$ représente un hydrogène, alcoyle inférieur, alcoxy inférieur, halogène ou trifluorométhyle; Ar représente un radical phényle, qui est substitué par un ou deux substituants du groupe alcoyle inférieur, alcoxy inférieur, alcényloxy inférieur, alcynyloxy-inférieur, halogène ou trifluorométhyle ou par un alcoylène inférieur-dioxy; et X représente O ou S, où les groupes désignés par "inférieur" contiennent au plus 7 atomes de carbone, et leurs sels d'addition d'acide.

4. Procédé selon la revendication 1, caractérisé en ce qu'on prépare des composés de formule IA présentés dans la revendication 3, où $R_1$ et $R_2$ représentent indépendamment l'un de l'autre un hydrogène ou un alcoyle inférieur, ou $R_1$ et $R_2$ représentent ensemble avec l'atome d'azote un pyrrolidino ou plpéridino; $R_3$, Ar et X ont les significations données dans la revendlcation 3, où les groupes désignés par "inférieur" contiennent au plus 7 atomes de carbone, et leurs sels d'addition d'acide.

5. Procédé selon la revendication 1, caractérisé en ce qu'on prépare des composés de formule 1B

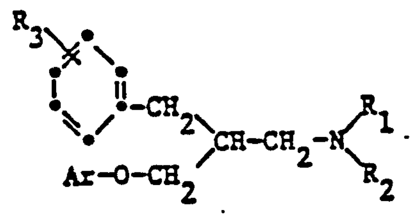

(IB),

où $R_1$ et $R_2$ représentent indépendamment l'un de l'autre un hydrogène ou un alcoyle inférieur; $R_3$ représente un hydrogène, un halogène ou un trifluorométhyle; Ar représente un phényle, qui est substitué par 1 ou 2 substituants identiques ou différents du groupe alcoyle inférieur, alcoxy inférieur, alcényloxy inférieur, alcoyle inférieur-mercapto, alcoyle inférieur-sulfinyle, alcoyle inférieur-sulfonyle, halogène ou trifluorométhyle, où les groupes désignés par "inférieur" contiennent au plus 7 atomes de carbone; et leurs sels d'addition d'acide.

6. Procédé selon la revendication 1, caractérisé en ce qu'on prépare des composés de formule I donnés dans la revendicatlon 1, où $R_1$ représente un hydrogène ou un méthyle, $R_2$ représente un méthyle; $R_3$ représente un hydrogène, alcoyle inférieur, alcoxy inférieur, halogène ou trifluorométhyle; n représente le nombre entier 1 ou 2, X représente un oxygène et Ar un phényle non substitué, où les groupes désignés par "inférieur" contiennent au plus 7 atomes de carbone, et leurs sels d'addition d'acide.

7. Procédé selon la revendication 1, caractérisé en ce qu'on prépare la N,N-diméthyl-2-benzyl-3-(4-trifluorométhylphénoxy)propylamine et ses sels d'addition d'acide.

8. Procédé selon la revendication 1, caractérisé en ce qu'on prépare la N,N-diméthyl-2-(3-chlorobenzyl)-3-(4-méthylmercaptophénoxy)propylamine et ses sels d'addition d'acide.

9. Procédé selon la revendication 1, caractérisé en ce qu'on prépare la N-méthyl-2-benzyl-3-(4-trifluoro-méthylphénoxy)propylamine et ses sels d'addition d'acide.

10. Procédé selon la revendication 1, de préparation de composés de formule IA présentée dans la revendication 3, où $R_1$ et $R_2$ représentent indépendamment l'un de l'autre un hydrogène ou un alcoyle inférieur, ou $R_1$ et $R_2$ représentent avec l'atome d'azote un pipéridino ou pyrrolidino, ou un noyau hétérocyclique saturé à 5 ou 6 chaînons, qui contient NH, N-alcoyle inférieur, O ou S; $R_3$ représente un hydrogène, alcoyle inférieur,

alcoxy inférieur, halogène ou trifluorométhyle; Ar représente un radical phényle qui est substitué par un ou deux substituants du groupe alcoyle inférieur, alcoxy inférieur, alcényloxy inférieur, alcynyloxy inférieur, alcoyle inférieur-mercapto, alcoyle inférieur-sulfinyle, alcoyle inférieur-sulfonyle, halogène ou trifluorométhyle ou par un alcoylène inférieur-dioxy; et X représente O ou S, où les groupes designés par "inférieur" contiennent au plus 7 atomes de carbone, leurs N-oxydes, leurs sels d'addition d'acide et leurs sels quaternaires de N-alcoyle inférieur ou N-benzyle, caractérisé en ce que

a) on condense un composé de formule II

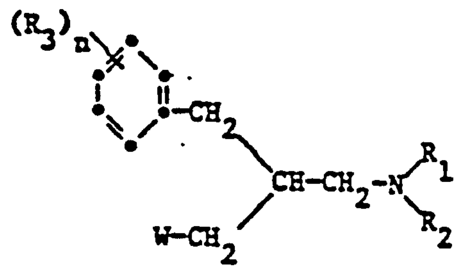

(II)

ou un de ses sels d'addition d'acide, avec un composé de formule III

Ar -Y (III)

où $R_1$, $R_2$, $R_3$, n et Ar ont les significations données ci-dessus, l'un des symboles W et Y représente le groupe -XH ou XH sous la forme d'un sel métallique, où X a la signification donnée ci-dessus, et l'autre représente le groupe XH sous forme estérifiée réactive ou sous la forme d'un dérivé d'iso-urée ou d'isothiourée N,N'-disubstitué ou

b) on alcoyle une amine de formule IV

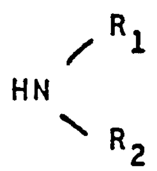

(IV)

ou un de ses sels d'addition d'acide, avec un composé de formule V

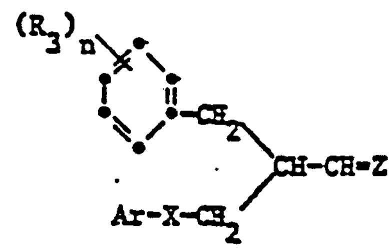

(V),

où Z représente un atome d'hydrogène avec un groupe hydroxy estérifié réactif ou le groupe oxo, et Ar, X, $R_3$ et n ont les significations données ci-dessus, et lorsque le groupe Z représente un oxo, l'alcoylation est conduite dans des conditions réductrices, ou

c) on réduit un composé de structure qui est analogue à celle du composé de formule I défini ci-dessus, et qui présente au moins un groupe oxo sur un atome de carbone voisin de l'atome d'azote d'amino, ou

d) on sature avec de l'hydrogène la ou les liaisons multiples dans un composé de structure qui est analogue à celle du composé de formule I défini ci-dessus, et où dans la partie aliphatique de la molécule, y compris le groupe amino, on trouve une ou deux doubles liaisons, ou

e) on sépare le groupe $R_0$ dans un composé de formule VI

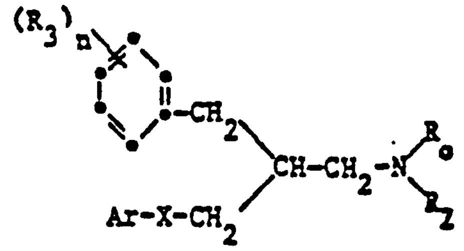

(VI),

où $R_2$, $R_3$, X, n et Ar ont les significations données ci-dessus, et $R_0$ représente un groupe protecteur d'amino, et l'on obtient un composé de formule I où $R_1$ est un hydrogène ou

f) on réduit un composé de formule VII

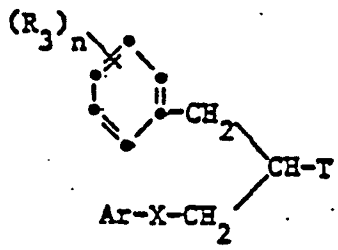

(VII),

où T représente un cyano, $R_3$, n, X et Ar ont les significations données ci-dessus, et l'on obtient un composé de formule I où chacun des symboles $R_1$ et $R_2$ représente un hydrogène, si nécessaire on protège temporairement dans tous ces procédes un groupe gênant, et l'on isole le composé de formule I obtenu, et/ou, si on le désire, on transforme un composé de formule I obtenu en un autre composé de formule I, et/ou, si on le désire, on transforme un composé libre obtenu en un sel ou un sel obtenu en le composé libre ou en un autre sel, et/ou, si on le désire, on dédouble un mélange d'isomères ou de racémates obtenu en les isomères ou racémates isolés, et/ou, si on le désire, on dédouble les racémates obtenus en les antipodes optiques.

11. Procédé selon la revendication 10, caractérisé en ce qu'on prépare des composés de formule IA présentés dans la revendication 10, où $R_1$ et $R_2$ représentent indépendamment l'un de l'autre un hydrogène ou un alcoyle inférieur ayant de 1 à 7 atomes de carbone où $R_1$ et $R_2$ représentent avec l'atome d'azote un pyrrolidino ou un pipéridino, $R_3$, Ar et X ont les significations données dans la revendication 10; et leurs sels d'addition d'acide.

12. Procédé selon la revendication 10, caractérisé en ce qu'on prépare des composés de formule IB

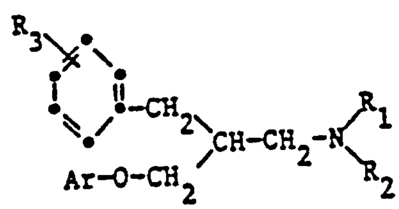

(IB),

où $R_1$ et $R_2$ représentent indépendamment l'un de l'autre un hydrogène ou un alcoyle inférieur; $R_3$ représente un hydrogène, un halogène ou un trifluorométhyle; Ar représente un phényle, qui est substitué par 1 ou 2 substituants identiques ou différents du groupe alcoyle inférieur, alcoxy inférieur, alcényloxy inférieur, alcoyle inférieur-mercapto, alcoyle inférieur-sulfinyle, alcoyle inférieur-sulfonyle, halogène ou trifluorométhyle, où les

groupes désignes par "inférieur" contiennent au plus 7 atomes de carbone, et leurs sels d'addition d'acide.

13. Procédé selon la revendication 10, caractérisé en ce qu'on prépare la N,N-diméthyl-2-benzyl-3-(4-trifluorométhylphénoxy)propylamine et ses sels d'addition d'acide.

14. Procédé selon la revendication 10, caractérisé en ce qu'on prépare la N,N-dimethyl-2-(3-chlorobenzyl)-3-(4-méthylmercaptophénoxy)propylamine et ses sels d'addition d'acide.

15. Procédé selon la revendication 10, caractérisé en ce qu'on prépare la N-méthyl-2-benzyl-3-(4-trifluorométhylphénoxy)propylamine et ses sels d'addition d'acide.

16. Procédé de préparation d'une préparation pharmaceutique, caractérisé par l'administration d'une substance active selon l'invention selon l'une des revendications 1 à 9, avec un support pharmaceutique.

17. Procédé de préparation d'une préparation pharmaceutique, caractérisé par l'administration d'une substance active selon l'invention selon l'une des revendications 10 à 15, avec un support pharmaceutique.